# EUROPEAN PATENT SPECIFICATION

(11) **EP 1 337 242 B1**
(45) Date of publication and mention of the grant of the patent: **30.03.2011**
(21) Application number: 01999202.3
(22) Date of filing: 19.10.2001
(51) Int. Cl.: A61K 9/14, A61K 48/00, C12N 15/63

(54) **GENE DELIVERY FORMULATIONS FOR TREATMENT OF ISCHEMIC CONDITIONS**
GEN-ABGABEFORMULIERUNGEN ZUR BEHANDLUNG ISCHÄMISCHER ZUSTÄNDE
PREPARATIONS DE DIFFUSION DE GENES POUR LE TRAITEMENT DE PATHOLOGIES ISCHEMIQUES

(30) Priority: 20.10.2000 US 242277 P; 29.05.2001 US 294454 P
(43) Date of publication of application: 27.08.2003
(60) Divisional application: 10183865.4
(73) Proprietor: VICAL INCORPORATED, San Diego, California 92121-4340 (US)
(72) Inventor: COLEMAN, Michael, E., The Woodlands, TX 77381 (US); MACLAUGHLIN, Fiona, 2010 Branard Houston, TX 77098 (US); WANG, Jijun, Pearland, TX 77584 (US); THIESSE, Mary, L., Houston, TX 77067 (US); YOUNG, Stewart, Portola Valley, CA 94208 (US); NORDSTROM, Jeffrey, L., College Station, TX 77840 (US)
(74) Representative: Williams, Richard Andrew Norman
(86) International application number: PCT/US2001/051307
(87) International publication number: WO 2002/061040

(56) References cited:
- WO-A-00/47186
- WO-A2-00/06759
- WO-A2-00/09086
- WO-A2-01/65911
- WO-A2-96/21470
- FR-A- 2 759 298
- US-A- 5 616 487
- US-A- 5 877 281
- US-A- 5 945 100
- US-A- 6 080 728
- US-E- R E36 665
- LOSORDO D W ET AL: "GENE THERAPY FOR MYOCARDIAL ANGIOGENESIS INITIAL CLINICAL RESULTS WITH DIRECT MYOCARDIAL INJECTION OF PHVEGF165 AS SOLE THERAPY FOR MYOCARDIAL ISCHEMIA" CIRCULATION, AMERICAN HEART ASSOCIATION, DALLAS, TX, US, vol. 98, no. 25, 22 December 1998 (1998-12-22), pages 2800-2804, XP000946361 ISSN: 0009-7322
- MUMPER R J ET AL: "Protective interactive noncondensing (PINC) polymers for enhanced plasmid distribution and expression in rat skeletal muscle" JOURNAL OF CONTROLLED RELEASE, ELSEVIER SCIENCE PUBLISHERS B.V. AMSTERDAM, NL, vol. 52, no. 1-2, 2 March 1998 (1998-03-02), pages 191-203, XP004113667 ISSN: 0168-3659
- JANG JAMES J ET AL: "Developmentally regulated endothelial cell locus: A novel angiogenic protein" JOURNAL OF THE AMERICAN COLLEGE OF CARDIOLOGY, vol. 33, no. 2 SUPPL. A, February 1999 (1999-02), page 250A, XP008053613 & 48TH ANNUAL SCIENTIFIC SESSION OF THE AMERICAN COLLEGE OF CARDIOLOGY; NEW ORLEANS, LOUISIANA, USA; MARCH 7-10, 1999 ISSN: 0735-1097
- JANG JAMES J ET AL: "Developmentally regulated endothelial cell-locus-1 (Del1): A novel angiogenic protein; Its role in ischemia" CIRCULATION, vol. 100, no. 18 SUPPL., 2 November 1999 (1999-11-02), page I.58, XP008053454 & 72ND SCIENTIFIC SESSIONS OF THE AMERICAN HEART ASSOCIATION; ATLANTA, GEORGIA, USA; NOVEMBER 7-10, 1999 ISSN: 0009-7322
- LIU F ET AL: "EFFECT OF NON-IONIC SURFACTANTS ON THE FORMATION OF DNA/EMULSION COMPLEXES AND EMULSION-MEDIATED GENE TRANSFER" PHARMACEUTICAL RESEARCH, NEW YORK, NY, US, vol. 13, no. 11, November 1996 (1996-11), pages 1642-1646, XP001012538 ISSN: 0724-8741

## Description

### Background Of The Invention

### Field Of Invention

This invention relates to stimulating angiogeriesis through the delivery and expression of nucleic acids encoding angiogenic factors. In particular, the formulations have applicability to the amelioration of ischemic conditions in peripheral vascular and coronary artery disease.

### Description of Related Art

Ischemia is a medical term describing a shortage of blood supply to an organ or tissue of the body. Ischemia typically results from narrowing or obstruction in the arteries that supply oxygen-rich blood to the tissues. Severe and prolonged ischemia leads to death of the affected tissue (infarction).

Coronary artery disease (CAD) refers to diseases of the blood vessels supplying oxygenated blood to the musculature of the heart (myocardium) resulting in cardiac ischemia. Narrowing or occlusion of one or more of the coronary arteries results in cardiac ischemia. Transient ischemia resulting from a failure of the blood supply to meet demands placed on the heart by increased physical activity or other stress results in angina or chest pain. Severe or total obstruction of blood flow may result in death of heart muscle commonly referred to as a myocardial infarction (heart attack). Heart disease is the leading cause of death in the United States. Cardiac ischemia is currently treated through the use of medication and physical conditioning to reduce the heart's oxygen demands or with drugs, angioplasty or bypass surgery to improve blood flow to the heart.

Peripheral vascular disease (PVD) refers to diseases of blood vessels outside the heart and brain. Narrowing of the vessels that carry blood to leg and arm muscles is a typical cause of PVD with single or multiple stenosis and/or occlusion of the iliac-femoral-popliteal arterial axis determining a reduction of the perfusion of the muscles and the skin of the lower limbs and thus a progressive tissue ischemia. Peripheral artery disease (PAD) is a condition similar to coronary artery disease and carotid artery disease.

In PAD fatty deposits build up along artery walls and affect blood circulation, primarily in arteries leading to the legs and feet. Atherosclerosis is the most common cause of chronic arterial occlusive disease of the lower extremities and can lead to clinical conditions ranging from intermittent claudication (ischemic pain) to ulceration and gangrene. The arterial narrowing or obstruction that occurs as a result of the atherosclerotic process reduces blood flow to the lower limb during exercise or at rest. A spectrum of symptoms results, the severity of which depends on the extent of the involvement and the available collateral circulation. The superficial femoral and popliteal arteries are the vessels most commonly affected by the atherosclerotic process. The distal aorta and its bifurcation into the two iliac arteries are the next most frequent sites of involvement.

Peripheral Arterial Disease (PAD) accounts for a sizable portion of annual health-care expenditures. Furthermore, beyond the actual health-care dollars spent, PAD is a major cause of disability, loss of work/wages, and lifestyle limitations (Rosenfield, K., and Isner, JM. (1998). In: Comprehensive Cardiology Medicine. J. Topol, ed. Lippincott-Raven Publishers, Philadelphia 3109-3134.) It has been estimated that PAD affects 1 in 20 people over the age of 50 or 8 million people in the United States, being more commonly diagnosed in men than in women (Creager, MA. (2001) Cardiol Rev. 9, 238-245)

Treatments for PVD include nonsurgical measures such as exercise, risk factor modification, and pharmacological therapy, as well as surgical treatment, which includes interventional radiological procedures such as angioplasty or stent insertion and surgical treatment such as endarterectomy, bypass grafting, and amputation. Angioplasty involves passage of a catheter with a deflated balloon on its tip into the narrowed artery segment, inflation of the balloon, and widening of the narrowed segment. However, no effective pharmacological treatment is available for vascularisation defects in the lower limbs. Many patients presenting with persistant ischaemic ulcers are not suitable for surgical or endovascular approaches.

In response to insufficiency of perfusion of the heart in CVD, the vascular bed may develop additional blood vessels, called collaterals that serve to route blood around areas of coronary narrowing and thus perfuse the myocardium. In the treatment of myocardial and peripheral ischemia, the induction of angiogenesis or new blood vessel growth would be expected to increase perfusion of ischemic tissues.

Studies have recently established the feasibility of using recombinant angiogenic growth factors, such as fibroblast growth factor (FGF) family (Yanagisawa-Miwa, et al., Science, 257:1401-1403 (1992) and Baffour, et al., J Vasc Surg, 16:181-91 (1992)), endothelial cell growth factor (ECGF)(Pu, et al., J Surg Res, 54:575-83 (1993)), vascular endothelial growth factor (VEGF) (Takeshita, et al., Circulation, 90:228-234 (1994) and Takeshita, et al., J Clin Invest, 93:662-70 (1994)) and angiopoietin-1 in combination with VEGF (Chae, J.K. et al. Artherioscler Thromb Vasc Biol. December 2000) to encourage collateral artery development in animal models of myocardial and hindlimb ischemia. Repeated intra-muscular administration of growth factor was required to maintain an optimally high and local concentration. The requirement for repeated administration is a major limitation of recombinant protein therapy. Repeated administration of recombinant proteins produces spikes of protein concentration with only intermittent concentrations in the therapeutic range. Recent results from phase II trials of recombinant VEGF₁₆₅ and bFGF proteins in patients with myocardial ischemia have been either negative at 3 months or equivocal at 1 year suggesting that the short tissue residence time obtained with recombinant protein therapy is insufficient to obtain the desired therapeutic effect. (Chiron Corp. Press Release 3/12/2000; http://www.prnewswire.com/CHIR; Henry, TD. et al. (1998) J. Am. Coll. Cardiol. 31, 65A.)

Gene therapy has recently provided the pharmacological arts with the ability to deliver functional recombinant genes carried in expression constructs capable of mediating expression of these genes in host cells in vivo. Instead of delivering purified or recombinant proteins to a patient, delivery of genes in the context of expression control elements permits the local production of proteins by the patient. Production of proteins in vivo by the patient emulates natural expression and can result in prolonged local production of desired proteins in therapeutic concentration from a single administration.

Arterial gene transfer constitutes an alternative strategy for accomplishing therapeutic angiogenesis in patient with limb ischemia (Kanno, S. et al. (1999). Circulation 99, 2682-2687; Liau, G. et al. (2001) Drug Disc Today 1, 689-697; Rissanen, TT. et al. (2001) Eur. J. Clin. Invest. 31, 651-666). The potential requirement to maintain a suitably high and local concentration over a period of days or weeks constitutes and advantage for gene transfer versus recombinant protein therapy (Isner JM. and Asahara T. (1999) J. Clin. Invest. 103,1231,-1236. Various angioigenic approaches of this nature are already being investigated in clinical trials (Carmeliet, P. and Jain, RK. (2000). Nature 407, 249-257).

Delivery of angiogenic factors as a gene therapy offers the potential for enhanced efficacy, less frequent dosing, and reduced systemic toxicity versus therapy with the recombinant protein. However, effective gene therapy requires identification of proteins with a desired therapeutic profile, the generation of expression vectors able to control production of genes encoding the desired protein, and efficient localized delivery of the expression vector to cells able to express the desired protein without causing local tissue destruction or systemic reaction.

A gene therapy for PVD by delivery of DNA encoding vascular endothelial growth factor (VEGF) for the promotion of angiogenesis has been tested in a human clinical trial. However, lower-extremity edema was observed in 31 of 90 (34%) patients treated indicating that VEGF expression may have the undesirable side effect of increased vascular permeability. (Baumgartner et al. Ann Intern Med (2000) 132(11):880-4). Recent reports have described an increase in blood flow in infarcted myocardium by the overexpression of hepatocyte growth factor (HGF) from a gene delivered using hemagglutinating virus of Japan (HVJ)-coated liposomes delivered by direct injection into the myocardium, direct infusion into a coronary artery as well as incubation within the pericardium. (Aoki et al. Gene Therapy 7 (5), 417-427, 2000.)

Delivery methods or vehicles that can deliver genes efficiently without concomitant tissue injury is critical to effective gene therapy. Delivery of genes to the vascular tissue in vivo using viral vectors, including through the use of poloxamer gel formulation to restrict movement of the viral formulation from the site of administration, has been described. (Feldman et al. Gene Therapy (1997) 4, 189-198; Van Belle et al. Human Gene Therapy (1998) 9, 1013-1024; Hammond et al., US Patent No. 6,100,242). Delivery of non-viral naked DNA to the heart using direct injection was described by Wolff et al., US Patent No. 5,693,622. Leiden et al described delivery of genes to the heart using direct injection in US Patent No. 5,661,133. Viral delivery to the myocardium using selective pressure regulated retroinfusion of coronary veins has also been described. Boekstaegers et al. Gene Therapy (2000) 7, 232-240. However, viral vectors suffer from several critical disadvantages. Viral vectors must be grown in mammalian culture and may be contaminated with other viruses originating from the host cells or required media components such as calf serum. Viral vectors necessarily involve the introduction of foreign viral proteins. Administration of such vectors in vivo can result in profound immune responses in the host. In the prior art publication WO 96/21470, Poloxamer 407 is used for gene delivery in vitro, and polyvinylpyrrolidone is used for gene delivery in vivo.

The field of angiogenic growth factor therapy for patients with advanced ischemic heart disease has progressed rapidly over the last five years. (Simons et al. Circulation (2000) 102:e73-e86). Despite recent advances in therapeutic modalities for treatment of ischemia, there remains a further need for the identification of a protein that can be administered as a gene that is able to promote collateral vessel growth in ischemic tissue in mammals without undesirable side effects. Further, delivery formulations are needed that can deliver such genes to ischemic tissue with increased efficiency but without immediate local or systemic toxicity or the generation of pathologic immune responses.

### Summary Of The Invention

The present inventors have developed a novel approach for efficient delivery of angiogenic factors to the cardiac and peripheral vasculature that avoids problems with toxicity inherent to existing delivery technologies.

In one embodiment, a polymeric gene delivery system provides protection of the angiogenic factor expression plasmid from degradation by extracellular nucleases and facilitates its uptake by skeletal and cardiac myofibers within the delivery region.

A formulated expression system for efficient delivery of a gene encoding the novel angiogenic factor Developmental Endothelial Locus (Del-1) protein is provided.

A formulated DEL-1 encoding plasmid DNA is provided that enables sustained, local expression of Del-1 in a manner that more closely mimics the autocrine/paracrine modes of action associated with endogenous Del-1. Local delivery can reduce systemic exposure and potential toxicities associated with systemic administration.

A non-viral plasmid-based Del-1 gene formulation is disclosed that provides for localized treatment of ischemia in a peripheral limb, in cardiac muscle, in the kidney associated with renal vascular disease, ischemia associated with cerebral vascular disease, wound healing, non-union fractures associated with ischemia and avascular necrosis of the femoral head. The Del-1 gene is administered by intramuscular injection, intravascular, intracapsular into the joint, or by retrograde venous perfusion. In one embodiment the retrograde venous perfusion is of a limb, kidney, liver, brain, or heart.

In one embodiment, the formulated Del-1 expression system is comprised of a plasmid expression system formulated with a poloxamer polymeric gene delivery system. The plasmid expression system contains an eukaryotic expression cassette encoding the full length human Developmental Endothelial Locus (Del) 1 protein.

In one embodiment, the formulated Del-1 expression system is provided as a single vial formulation that is stable at 2-8° C. Treatment of the ischemic tissue with the formulated Del-1 expression system can be repeated if necessary.

Del-1 is a ligand for the alpha-v-beta-3 (avb3) integrin receptor. Thus, in another embodiment Del-1 is used in combination with another growth factor that acts as a ligand for a different receptor known to be important in the development of new blood vessels. In one embodiment, the nucleic acid encoding Del-1 is delivered in conjunction with a nucleic acid encoding VEGF, which is a ligand for the vascular endothelial growth factor-2 (VEGF-2) receptor, for the modulation of angiogenesis and vasculogenesis. The use of the Del-1 and VEGF genes in combination addresses several unmet medical needs including coronary artery disease, wound healing, peripheral artery disease, and rheumatoid arthritis.

### Brief Description Of The Drawings

A better understanding of this invention can be obtained when the following detailed description of the preferred embodiments is considered in conjunction with the following drawings.
**Figure 1****.** Effect of plasmid and poloxamer 188 concentration on delivery of expression plasmid in murine skeletal muscle.
**Figure 2****.** Expression of luciferase in tibialis anterior muscles of rats injected with luciferase expression plasmids formulated with isotonic saline compared with polymeric delivery systems.
**Figure 3a** **and b.** Expression plasmid maps for hDel-1.
**Figure 4****.** Expression of mDel-1 in tibialis anterior muscles of mice.
**Figure 5****.** Effects of Del-1 expression on capillary density in normoxic mouse skeletal muscle.
**Figure 6****.** Correlation of CD31 expression with expression of mDel-1 in normoxic tibialis anterior muscles of CD1 mice injected with different doses of formulated model-1 plasmid.
**Figure 7****.** Effects of hDel-1 plasmid on exercise tolerance following induction of hindlimb ischemia following ligation of the femoral artery.
**Figure 8****.** Effects of Del-1 and VEGF gene medicines in a rabbit model of hindlimb ischemia.
**Figure 9****.** Luciferase expression in murine myocardium following IM injection of formulated pLC1088 plasmid (10 microliters).
**Figure 10****.** Data shown represent luciferase expression in murine myocardium following direct intramyocardial injection (10 microliters).
**Figure 11a****.** Route of insertion of delivery catheter through the coronary sinus as viewed over the diaphragmatic aspect of the heart.
**Figure 11b****.** Placement of delivery catheter in the great cardiac vein as viewed over the sternocostal aspect of the heart.
**Figure 12****.** Depicts the sequence of human Del-1 (SEQ ID NO: 1) as utilized in the pDL1680 expression plasmid.
**Figure 13****.** Depicts the sequence of the pDL1680 human Del-1 expression plasmid (SEQ ID NO: 2).
**Figure 14****.** Depicts the increased reproducibility of expression with polymer based formulations.
**Figure 15****.** Depicts expression of hDel-1 mRNA within the myocardium of pigs treated by rIV delivery with either pDL1680 formulated in saline or pDL1680 formulated with 5 % poloxamer 188.
**Figure 16****.** Nucleic acid sequence of a codon optimized VEGF 165 (SEQ ID NO: 3).
**Figure 17****.** CD31 Staining at Day 7 for (A) control, (B) Del-1, (C) VEGF, and (D) Del-1/VEGF.
**Figure 18****.** Grid representing poloxamer and reverse poloxamer characteristics.
**Figure 19****.** Characteristics of useful poloxamers for muscle delivery.

### Detailed Description Of The Preferred Embodiments

The term "angiogenic protein" means any protein, polypeptide, mutein or portion that is capable of, directly or indirectly, inducing the formation of new blood vessels. Such proteins include, for example, developmental endothelial locus-1 (Del-1), acidic and basic fibroblast growth factors (aFGF and bFGF), vascular endothelial growth factor (VEGF), epidermal growth factor (EGF), transforming growth factor-alpha and -beta (TGF-alpha and TFG-beta), platelet-derived endothelial growth factor (PD-ECGF), platelet-derived growth factor (PDGF), tumor necrosis factor-alpha (TNF-alpha), hepatocyte growth factor (HGF), insulin like growth factor (IGF), erythropoietin, colony stimulating factor (CSF), macrophage-CSF (M-CSF), granulocyte/macrophage CSF (GM-CSF) and nitric oxide synthase (NOS). Preferably, the angiogenic protein contains a secretory signal sequence allowing for secretion of the protein. Del-1 and VEGF are preferred angiogenic proteins. The invention pertains to formulations of nucleic acids encoding del-1.

Developmentally Regulated Endothelial Locus-1 (Del-1) is a recently identified endothelial cell-specific extracellular matrix protein expressed during vascular development in the embryo. (Penta, K. et al (1999). J. Biol. Chem. 274, 11101-11109.) The Del-1 gene was identified in an enhancer trap in transgenic mice as a gene that is expressed primarily in the endothelium of the developing vasculature and in immediately adjacent cell types (Hidai, C., et al. (1998). Genes Dev. 1, 21-33.) Del-1 protein and nucleotide sequences encoding human and mouse Del-1 are the subject of U.S. Patent Nos. 5,877,281 and 5,874,562 incorporated herein by reference.

Further analysis of Del-1 expression in solid tumors and in acutely ischemic rodent skeletal muscle has indicated that expression of the Del-1 gene is locally up-regulated in areas of active angiogenesis. Del-1 is not normally expressed postnatally, but expression of Del-1 is up regulated in response to ischemia and other angiogenic stimuli. Del-1 has been shown to be involved in the migration and adherence of endothelial cells via ligation of the alpha-v, beta-3 integrin receptor. Recombinant Del-1 has been shown to increase vessel formation in the chorioallantoic membrane assays.

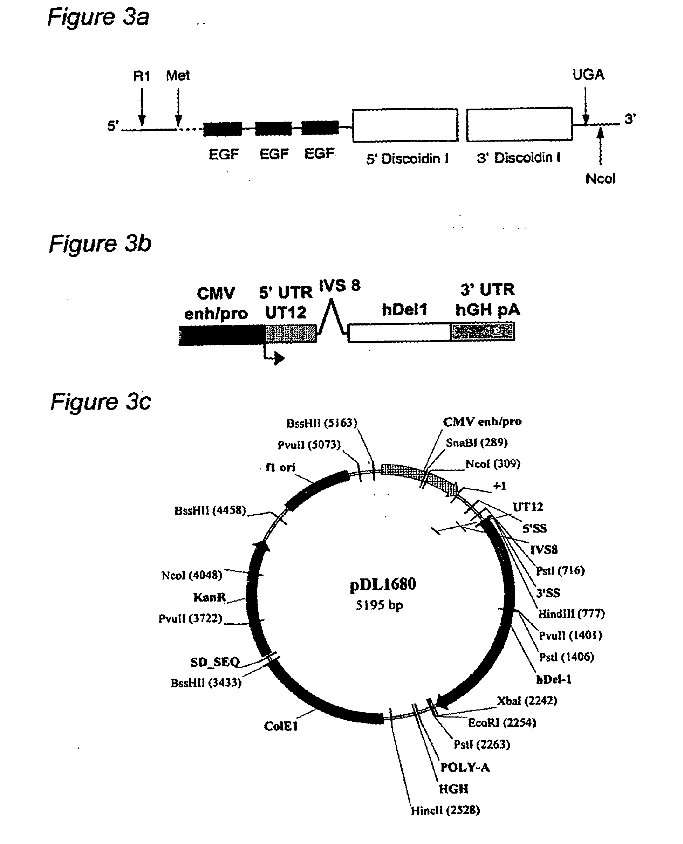

The full length Del-1 protein encoded by the Del-1 encoding plasmid is an approximately 52 kDa protein consisting of three EGF-like repeats followed by two discoidin I-like domains. An RGD sequence that mediates binding to the alpha-v, beta 3 ("αvB3" or "avb3") integrin receptor is contained within the B loop of the second EGF-like repeat in Del-1. The RGD sequence has been shown to be a cell binding site for fibronectin, discoidin I, nidogen/entactin, and tenascin (Anderson, 1990; Experientia 46:2). The overall structure of Del-1 is depicted in Figure 3a.

Available data indicates that the mechanism of action for Del-1 is unique among integrin receptor ligands and among known angiogenic factors. Ligand binding to the αvB3 integrin receptor provides an anti-apoptosis signal to ischemic endothelium (3) and is known to be requisite for angiogenesis (4). Given the complex structure of the Del-1 protein and the observation that C-terminal truncations of the full length Del-1 sequence support endothelial attachment, but do not elicit angiogenesis, it has been postulated that Del-1 may interact with a second, as yet unidentified, receptor (Penta et al., Journal of Biological Chemistry (1999) 274; 11101-11109).

The therapeutic potential of intramuscularly administered non-viral formulated Del-1 expression system has been shown by the present inventors in both the mouse and rabbit hindlimb ischemic model. Ischemia was created in the left hindlimb of 18 New Zealand White rabbits by resection of the femoral artery after ligating the distal external iliac artery. Baseline angiography was performed on the ischemic limb the day of surgery. On day three post-surgery, the left quadricep femoris muscle of the animals was injected with 5 mg of plasmid coding for VEGF, Del-1 or empty vector for a negative control. Thirty days post-treatment, angiography was performed again on the ischemic limb, and the quadricep femoris muscle from the treated limb was harvested and evaluated by RT-PCR, CD-31 immunoassay, and analysis of collateral vessels formed in the medial thigh of the rabbit by angiography. Also an ischemic model was induced in 32 CD-1 mice by ligating the distal internal iliac artery and the point of the femoral artery where it bifurcates into the deep femoral artery. The day of surgery 70 micrograms of plasmid coding for VEGF, Del-1 or empty vector was administered. A sham control group was also used in which the animal's hind limb was opened the same as in the surgeries and then closed again with no plasmid administered. The animals were run on a treadmill and their run time to exhaustion was recorded. The VEGF and Del-1 administration of non-viral formulated plasmid was significant (p<0.01) in the formation of new collateral vessels in the rabbit model. In the mouse model it was seen that the capillary to myocyte ratio increased 1.5 fold in the Del-1 group over controls which was significant at (p<0.05), and the run time to exhaustion on the treadmill was also significantly (p<0.05) improved.

In another embodiment, modulation of angiogenesis and vasculogenesis is achieved through generation of Del-1 in conjunction with another endothelial cell growth factor that utilizes a different receptor than that of Del-1. Del-1 is a ligand for the alpha-v-beta-3 (avb3) integrin receptor. (Hidai, C. et al. Genes and Development 1998, 12:21-33.).

In contrast, vascular endothelial growth factor, VEGF, is a ligand for the vascular endothelial growth factor receptor-1 (VEGFR-1, a.k.a. flt-1) and VEGFR-2 (a.k.a KDR in humans). Current data indicates signaling through flt-1 is primarily involved in endothelial cell migration and is not necessary for proliferation as is KDR. VEGFR-1 may actually antagonize the activity of VEGFR-2. (Yancopoulos, GD, et al. Nature (2000) 407: 242). The avb3 integrin receptor and the VEGF receptor-2 are involved in two significant pathways. It is believed that stimulation of the avb3 integrin receptor mediates endothelial cell migration, a process crucial to angiogenesis. The VEGF receptor-2 is a receptor tyrosine kinase that autophosphorylates upon stimulation, setting off a cascade of kinases that promote endothelial cell proliferation and motility. Stimulation of the VEGF-R2 results in up-regulation of avb3. Although avb3 is upregulated by ligation of VEGF-R2, it was possible that sufficient ligand to maximize the system was already present in the matrix such that further addition of Del-1 would not have an additive effect. The present inventors have now shown that addition of Del-1, a unique angiogenic integrin receptor ligand, is able to not only synergize with VEGF in promoting angiogenesis but results in a profound increase in endothelial cell migration and proliferation.

The present inventors have surprisingly found that delivery of the two genes at sub-maximal levels results in endothelial cell migration that is clearly superior to that of maximal doses of the genes individually. The two receptors have been previously identified to interact with each other upon stimulation, although neither requires direct binding with its ligand to bind with the other. However, stimulation of one receptor and subsequent binding of the unbound receptor leads to only partial binding. The basis of this invention states that co-administration of the two ligands, Del-1 and VEGF, allows for complete binding of the avb3 integrin and VEGF preceptor-2.

As used herein the term "Del-1 gene" means any DNA sequence encoding a functional protein of the Del-1 gene family. In accordance with the invention, any nucleotide sequence which encodes the amino acid sequence of the Del-1 gene product can be used to generate recombinant molecules which direct the expression of a Del-1 gene product. Due to the inherent degeneracy of the genetic code, other DNA sequences which encode substantially the same or a functionally equivalent amino acid sequence, may be used in the practice of the invention. Such DNA sequences include those that are capable of hybridizing to the murine and/or human Del-1 sequences under stringent conditions. The phrase "stringent conditions" as used herein refers to those hybridizing conditions that (1) employ low ionic strength and high temperature for washing, for example, 0.015M NaCl/0.0015M sodium citrate/0.1 % SDS at 50°C; (2) employ during hybridization a denaturing agent such as formamide, for example, 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C; or (3) employ 50% formamide, 5 x SSC (0.75M NaCl, 0.075M Sodium pyrophosphate, 5 x Denhardt's solution, sonicated salmon sperm DNA (50 g/ml), 0.1 % SDS, and 10% dextran sulfate at 42°C., with washes at 42°C. in 0.2 x SSC and 0.1% SDS. Altered DNA sequences which may be used in accordance with the invention include deletions, additions or substitutions of different nucleotide residues resulting in a sequence that encodes the same or a functionally equivalent gene product. The gene product itself may contain deletions, additions or substitutions of amino acid residues within a Del-1 sequence, which result in a silent change thus producing a functionally equivalent Del-1 protein. Such amino acid substitutions may be made on the basis of similarity in polarity, charge, solubility, hydrophobicity, hydrophilicity, and/or the amphipathic nature of the residues involved. For example, negatively charged amino acids include aspartic acid and glutamic acid; positively charged amino acids include lysine, histidine and arginine; amino acids with uncharged polar head groups having similar hydrophilicity values include the following: glycine, asparagine, glutamine, serine, threonine, tyrosine; and amino acids with nonpolar head groups include alanine, valine, isoleucine, leucine, phenylalanine, proline, methionine, tryptophan. The DNA sequences of the invention may be engineered in order to alter a Del-1 coding sequence for a variety of ends, including but not limited to, alterations which modify processing and expression of the gene product. For example, mutations may be introduced using techniques which are well known in the art, e.g., site-directed mutagenesis, to insert new restriction sites, to alter glycosylation patterns, phosphorylation, etc. In another embodiment of the invention, a Del-1 or a modified Del-1 sequence may be ligated to a heterologous sequence to encode a fusion protein. In order to express a biologically active Del-1, the nucleotide sequence coding for Del-1, or a functional equivalent, is inserted into an appropriate expression vector, i.e., a vector that contains the necessary elements for the transcription and translation of the inserted coding sequence.

As used herein the term "Del-1 protein" means any polypeptide sequence encoded by a nucleic acid derived from the sequence of the Del-1 gene and having Del-1 activity.

As used herein, vascular endothelial growth factor, ("VEGF"), is a homodimeric heavily glycosylated protein of 46-48 kDa (24 kDa subunits) although glycosylation is not required for biological activity. The homodimeric subunits are linked by disulphide bonds. The human gene has a length of approximately 12 kb and contains eight exons. At least four species of mRNA encoding VEGF-A have been identified and found to be expressed in a tissue-specific manner. The 165 amino acid form of the factor (VEGF-165) is the most common form found in most tissues. VEGF-121 and VEGF-165 are soluble secreted forms of the factor while VEGF-189 and VEGF-206 are mostly bound to heparin-containing proteoglycans on the cell surface or in the basement membrane. They arise from differential splicing with the 165 amino acid form of VEGF lacking sequences encoded by exon 6 and the 121 amino acid form lacking exon 6 and 7 sequences.

A high-affinity glycoprotein VEGF receptor of 170-235 kDa is expressed on vascular endothelial cells. The high-affinity receptor for VEGF, now known as VEGF-R1, has been identified as the gene product of the flt-1. Another receptor for VEGF, now known as VEGF-R2, is KDR, also known as flk-1. Signaling through the KDR/VEGFR-2 receptor up-regulates expression of integrin receptors.

Other VEGF-related factors are VEGF-B, which forms heterodimers with VEGF and VEGF-C (a.k.a. VEGF-2). VEGF-C is a protein of 23 kDa that is derived by proteolytic cleavage from a larger precursor. Another receptor for VEGF-C is Flt-4. VEGF-D has been described also and is the ligand for both KDR/Flk-1 and Flt-4. Thus, the ligands for KDR/VEGFR-2 include VEGF-A and VEGF-C and VEGF-D family members. (Yancopoulos, GD, et al. Nature (2000)). In one embodiment, VEGF-A, C and D are used in conjunction with Del-1 to promote angiogenesis.

By "suitable for internal administration" is meant that the compounds are suitable to be administered within the tissue of an organism, for example within a muscle. A delivery modality was originally described for delivery of drugs, cardioprotective agents or cardioplegia during myocardial surgery. (Kar et al. Heart Lung (1992) 21; 148-59; Herity et al. Catheter Cardiovasc Interv (2000) 51; 358-63). Retrograde delivery of naked plasmid DNA encoding the marker proteins LacZ and luciferase was described by Wolff in WO00/15285. No teaching or suggestion of DNA formulated with transfection facilitating agents was provided.

A "vector" is molecule incorporating nucleic acid sequences encoding therapeutic product(s) as well as, various regulatory elements for transcription, translation, transcript stability, replication, and other functions as are known in the art. A vector may be a nucleic acid such as a plasmid or other DNA vector. Alternatively, a vector may be modified virus whose native form contains the genomic material of a viral particle.

A "transcript stabilizer" is a sequence within the vector which contributes to prolonging the half life (slowing the elimination) of a transcript. "Post-translational processing" means modifications made to the expressed gene product. These may include addition of side chains such as carbohydrates, lipids, inorganic or organic compounds, the cleavage of targeting signals or propeptide elements, as well as the positioning of the gene product in a particular compartment of the cell such as the mitochondria, nucleus, or membranes. The vector may comprise one or more genes in a linear or circularized configuration. The vector may also comprise a plasmid backbone or other elements involved in the production, manufacture, or analysis of a gene product. An "expression vector" is a vector that allows for production of a product encoded for by a nucleic acid sequence contained in the vector. For example, expression of a particular growth factor protein encoded by a particular gene. A "gene product" means products encoded by the nucleic acid sequences of the vector.

By "prolong the localized bioavailability of a nucleic acid" is meant that a nucleic acid when administered to an organism in a composition comprising such a compound will be available for uptake by cells for a longer period of time than if administered in a composition without such a compound. This increased availability of nucleic acid to cells could occur, for example, due to increased duration of contact between the composition containing the nucleic acid and a cell or due to protection of the nucleic acid from attack by nucleases. The compounds that prolong the localized bioavailability of a nucleic acid are suitable for internal administration.

The compounds which prolong the localized bioavailability of a nucleic acid may also achieve one or more of the following effects, due to their physical, chemical or rheological properties: (1) Protect nucleic acid, for example plasmid DNA, from nucleases; (2) increase the area of contact between nucleic acid, such as plasmid DNA, through extracellular matrices and over cellular membranes, into which the nucleic acid is to be taken up; (3) concentrate nucleic acid, such as plasmid DNA, at cell surfaces due to water exclusion; (4) indirectly facilitate uptake of nucleic acid, such as plasmid DNA, by disrupting cellular membranes due to osmotic, hydrophobic or lytic effects. The following compounds may prolong the localized bioavailability of a nucleic acid: poloxamers (Pluronics®); poloxamines Tetronics); polyglutamate; ethylene vinyl acetates; polyethylene glycols; dextrans; polyvinylpyrrolidones; polyvinylalcohols; propylene glycols; and polyvinylacetates. These substances may be prepared as solutions, suspensions, gels, emulsions or microemulsions. By "solutions" is meant water soluble polymers and/or surfactants in solution with nucleic acids.

The compounds which prolong the bioavailability of a nucleic acid may also interact or associate with the nucleic acid by intermolecular forces and/or valence bonds such as: Van der Waals forces, ion-dipole interactions, ion-induced dipole interactions, hydrogen bonds, or ionic bonds. These interactions may serve the following functions: (1) stereoselectively protect nucleic acids from nucleases by shielding; (2) facilitate the cellular uptake of nucleic acid by "piggyback endocytosis". Piggyback endocytosis is the cellular uptake of a drug or other molecule complexed to a carrier that may be taken up by endocytosis. CV Uglea and C Dumitriu-Medvichi. Medical Applications of Synthetic Oligomers. In: Polymeric Biomaterials. Edited by Severian Dumitriu. Marcel Dekker, Inc. 1993, incorporated herein by reference. To achieve the desired effects set forth it is desirable, but not necessary, that the compounds which prolong the bioavailability of a nucleic acid have amphipathic properties; that is, have both hydrophilic and hydrophobic regions. The hydrophilic region of the compounds may associate with the largely ionic and hydrophilic regions of the nucleic acid, while the hydrophobic region of the compounds may act to retard diffusion of nucleic acid and to protect nucleic acid from nucleases. Additionally, the hydrophobic region may specifically interact with cell membranes, possibly facilitating endocytosis of the compound and thereby nucleic acid associated with the compound. This process may increase the pericellular concentration of nucleic acid. Agents which may have amphipathic properties and are generally regarded as being pharmaceutically acceptable are the following: poloxamers (Pluronics); poloxamines (Tetronics); ethylene vinyl acetates; polyethylene glycols; polyvinylpyrrolidones; polyvinylalcohols; and polyvinylacetates. Also, copolymer systems such as polyethylene glycol-polylactic acid (PEG-PLA), polyethylene glycol-polyhydroxybutyric acid (PEG-PHB), polyvinylpyrrolidone-polyvinylalcohol (PVP-PVA), and derivatized copolymers such as copolymers of N-vinyl purine (or pyrimidine) derivatives and N-vinylpyrrolidone.

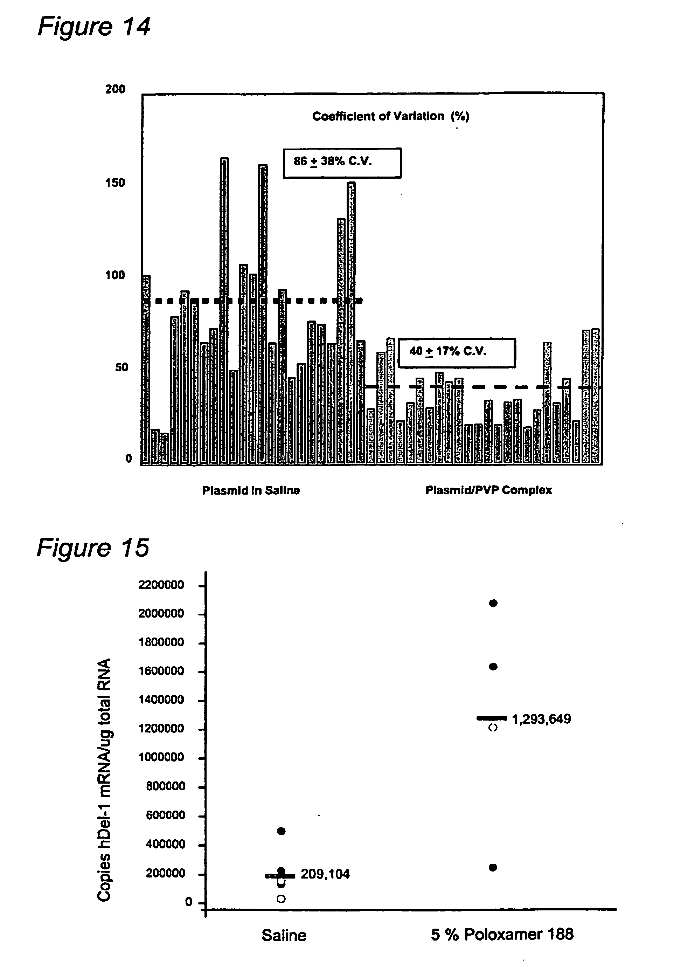

A particular advantage of non-condensing polymer formulations of non-viral nucleic acids is that their use greatly decreases the coefficient of variation as shown in Figure 14, comparing plasmid in saline and plasmid formulated with PVP, a non-condensing polymer.

As used herein term "poloxamer" means any di- or tri-block copolymer composed of the hydrophobe propylene oxide (POP, polyoxypropylene has the formula (C₃H_{6O})ₓ and thus has a unit mw of 58) and the hydrophile ethylene oxide (POE, polyoxyethylene has the formula (C₂H₄O)ₓ and thus has a unit mw of 44). Poloxamers are in the polyglycol chemical family. The common chemical name for poloxamers is polyoxypropylene-polyoxyethylene block copolymer. The CAS number is 9003-11-6.

Pluronic® is a trademark for poloxamers manufactured by BASF. In Europe the pharmaceutical grade poloxamers manufactured by BASF is sold under the mark Lutrol. Poloxamers of the Pluronic® type are tri-block copolymers in which the hydrophobe propylene oxide block is sandwiched between two hydrophile ethylene oxide blocks and has the following general formula and structure:

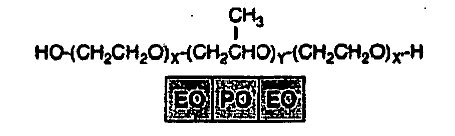

BASF poloxamers of the "reverse Pluronic®" type have the following structure:

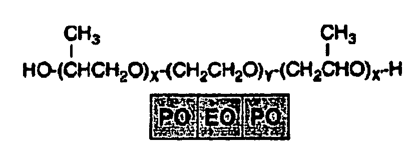

As used herein, the term "poloxamine" refers to poly(oxyethylene)-poly(oxypropylene) (POE-POP) block copolymers where a POE-POP unit is linked to another POE-POP unit by an amine and having the general structure (POEₙ - POPₘ)₂ -N - C₂H₄ - N - (POPₘ - POEₙ)₂. TETRONIC® and TETRONIC R nonionic surfactants produced by BASF are exemplary poloxamines. By virtue of their amine group, poloxamines have a positive charge but are not thought to condense DNA at the concentrations used.

Poloxamines are in the alkoxylated amine chemical family. Poloxamines of the BASF Tetronic® type have the chemical name: 1,2-Ethanediamine, polymer with the following formula: (POEₙ - POPₘ)₂ -N - C₂H₄ - N - (POPₘ - POEₙ)₂ and the CAS number: 11111-34-5. Reverse Tetronics® have the formula:
(POPₙ - POEₘ)₂ -N - C₂H₄ - N - (POEₘ - POPₙ)₂ and the CAS number: 26316-40-5.
TETRONIC® 904 is supplied as a liquid having an average molecular weight of 6,700 Da.
TETRONIC@ 908 is supplied as a solid having an average molecular weight of 25,000 Da.
TETRONIC@ 1107 is supplied as a solid having an average molecular weight of 15,000 Da.
TETRONIC@ 90R4 is supplied as a liquid having an average molecular weight of 7,240 Da.

### Example 1: Formulation for Enhanced Plasmid Delivery to Skeletal Myofibers

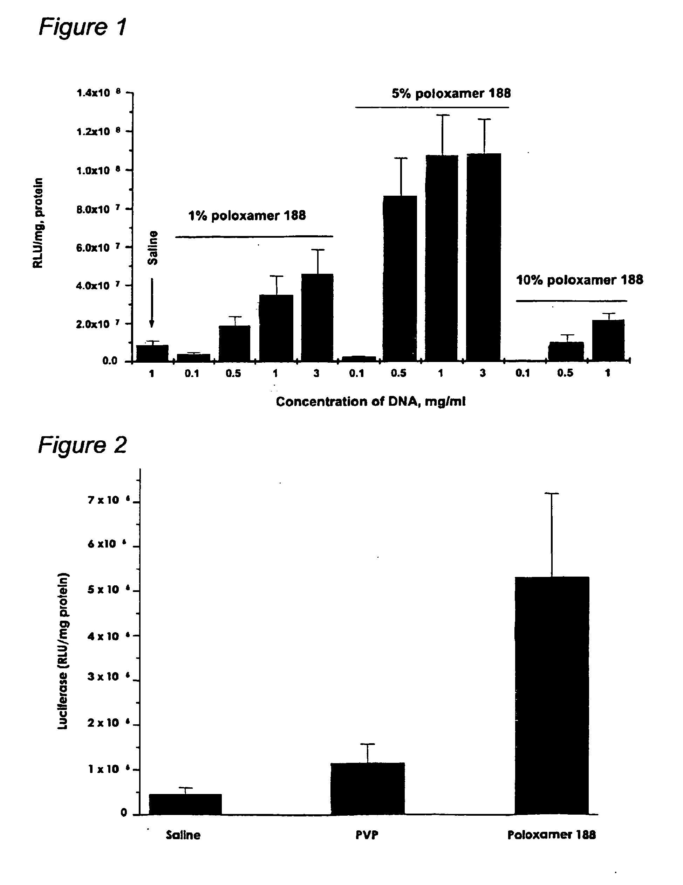

Enhanced delivery of plasmid to muscle may be accomplished by formulation with PINC™ (Protective, Interactive, Non-Condensing) polymeric delivery systems. The PINC™ delivery system for Del-1 is comprised of a U.S.P. NF grade poloxamer 188 (Pluronic® F68). Figure 1 shows the effect of plasmid and poloxamer 188 concentration on delivery of expression plasmid in murine skeletal muscle. Mice were injected im into the tibialis anterior muscles with 10 microliters plasmid/poloxamer 188 formulation containing plasmid and poloxamer at concentrations ranging from 0.1 to 3.0 mg/ml and 1 to 10 % (w/v), respectively. Injected muscles were harvested 7-day post injection and assayed for luciferase expression. Data presented are the mean +/- SEM for n = 10 muscles/group. An optimized plasmid/poloxamer 188 formulation consisting of 1 mg/ml plasmid with 5 % (w/v) poloxamer 188 yielded expression that was approximately one log higher than the level of expression observed when an equivalent concentration of plasmid was formulated in isotonic saline. This optimized plasmid poloxamer formulation was subsequently tested in rat skeletal muscle (Figure 2).

Figure 2 shows expression of luciferase in tibialis anterior muscles of rats injected with the luciferase expression plasmid pLC0888 formulated with isotonic saline, polyvinylpyrrolidone, or poloxamer 188 PINC™ delivery systems. The luciferase expression plasmid pLC0888 incorporates the cytomegalovirus (CMV) promoter, a 5' synthetic intron, a gene encoding luciferase, and the 3' polyadenylation signal and untranslated region from the bovine growth hormone gene. Bars with different superscripts are different (p<0.05). The approximate one log increase in gene expression over plasmid formulated in saline that was observed in mice was also observed in rats. Intramuscular injection of plasmid formulated in poloxamer 188 has been well tolerated, and has not been associated with any gross pathology.

In addition to formulation with non-condensing polymeric delivery systems, electroporation has also been utilized to enhance plasmid delivery to skeletal muscle in some studies. Electroporation using optimized parameters has been shown to yield an additional 1 to 2 log increases in plasmid delivery. This enhanced delivery results in expression of the plasmid-encoded transgene in a substantial majority of myofibers within limb muscle of mice. Electroporation has been used to investigate the full dose response to locally expressed Del-1. Results from preclinical studies indicate that therapeutic levels of Del-1 expression may be achieved with or without further mechanical enhancement such as with electroporation.

### Example 2: Del-1 Expression Plasmid

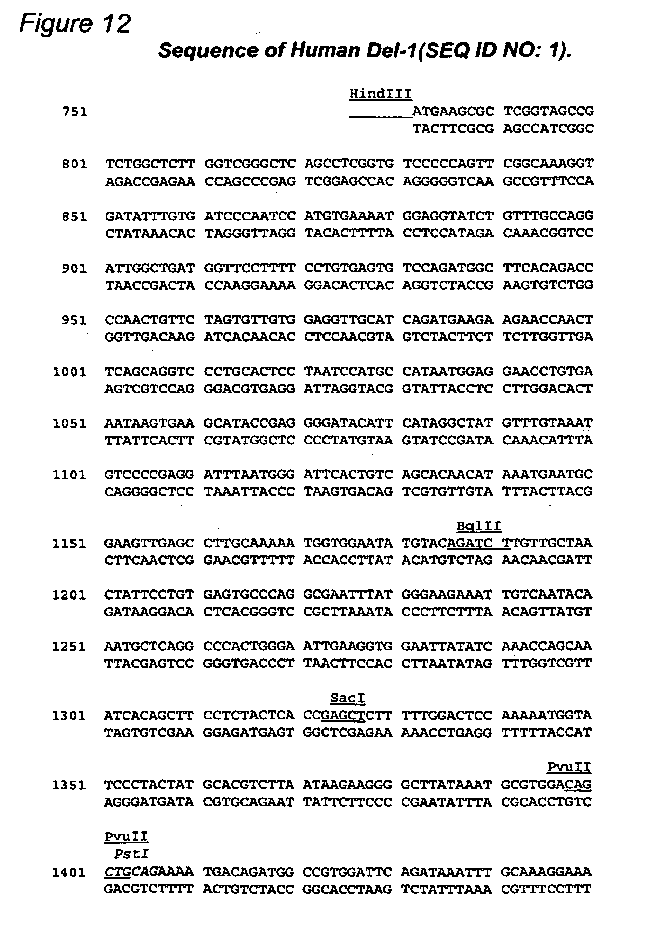

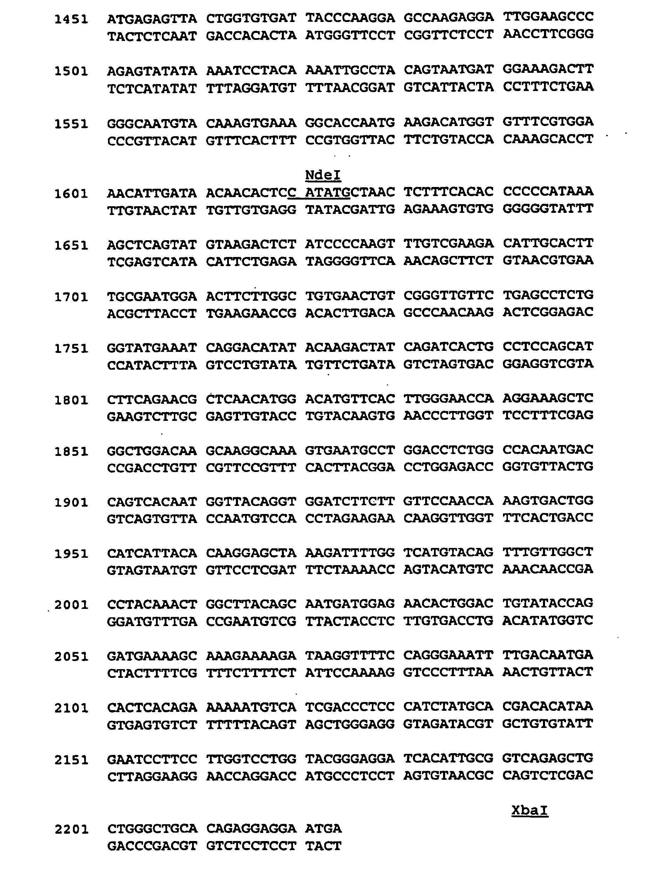

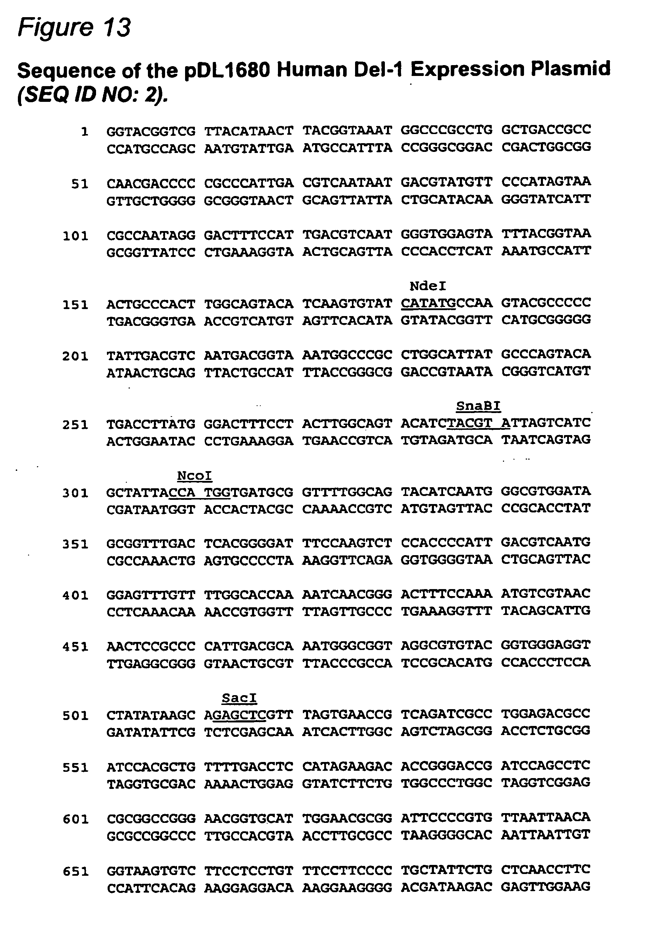

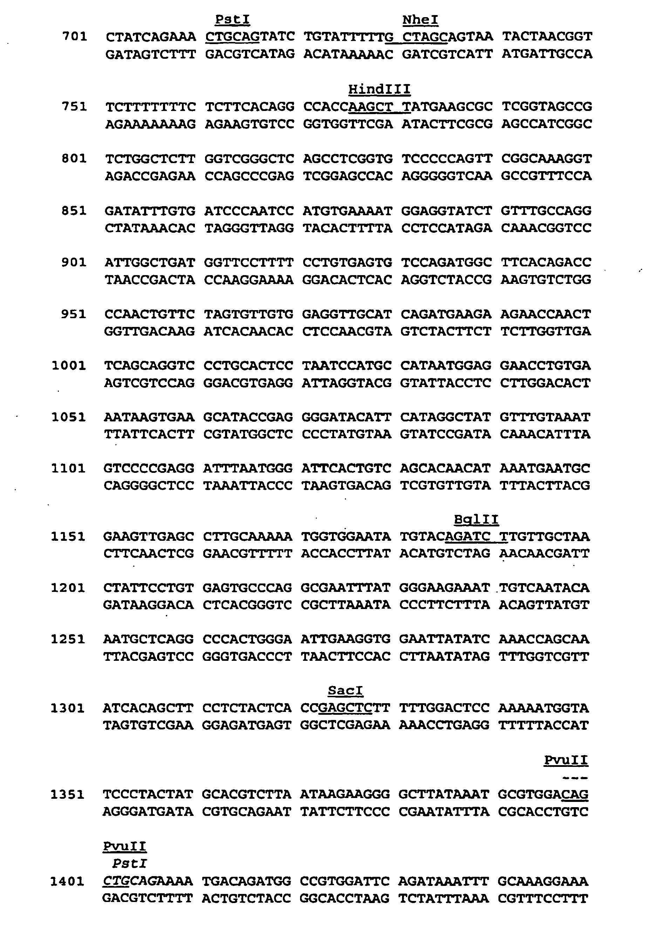

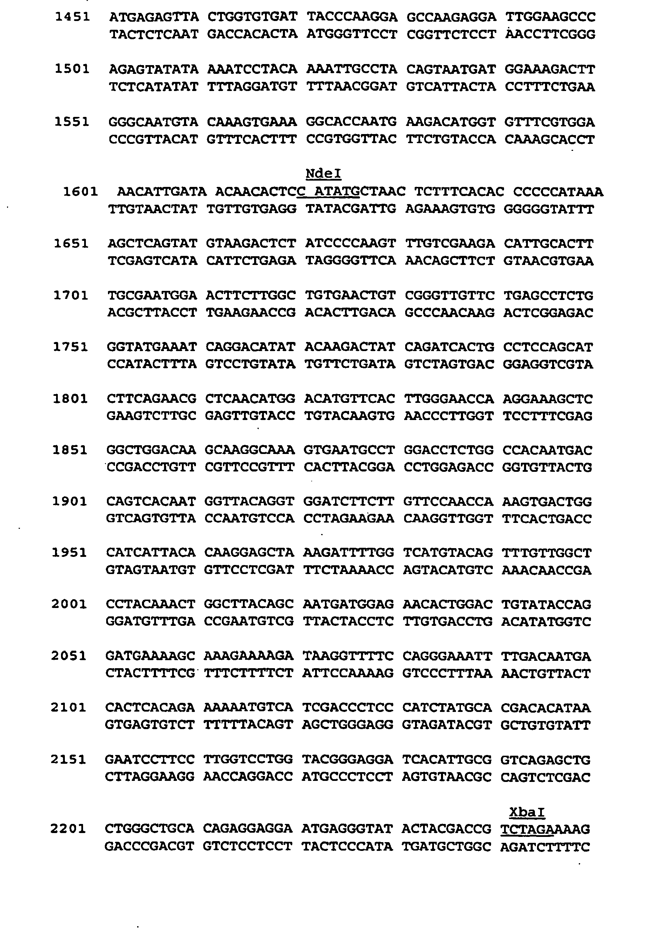

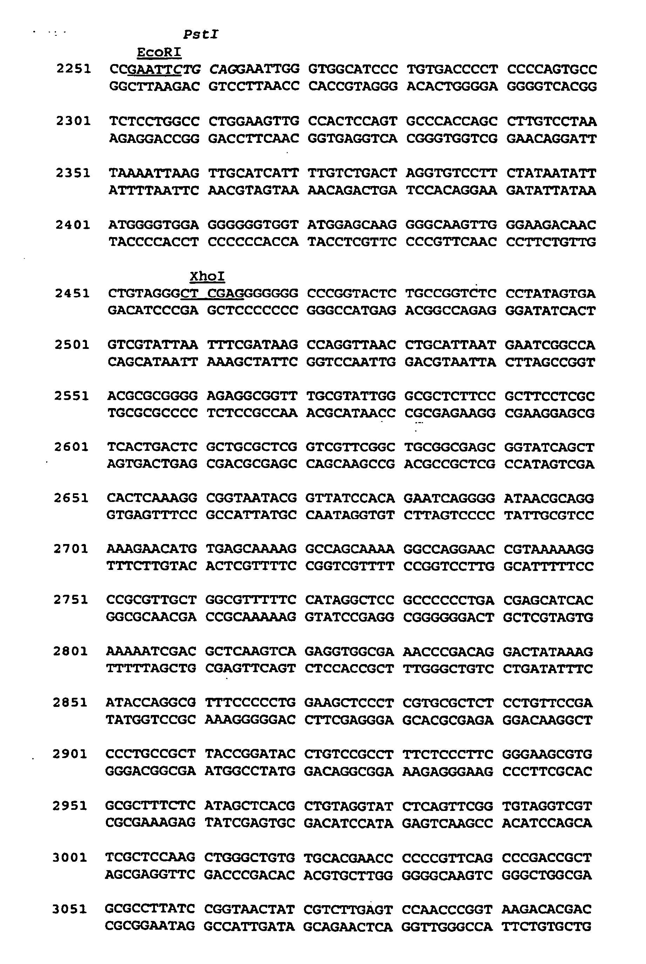

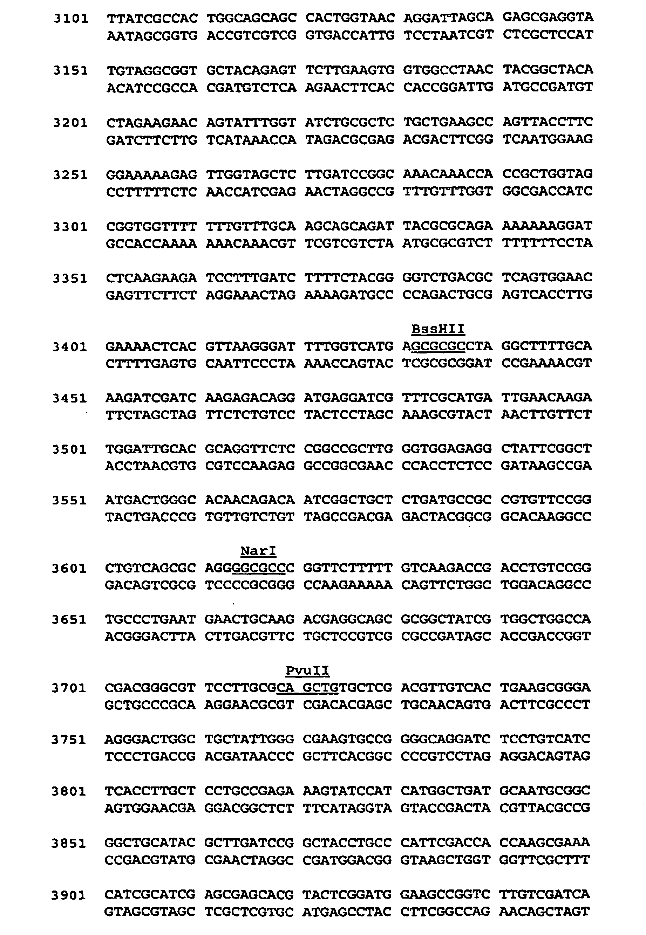

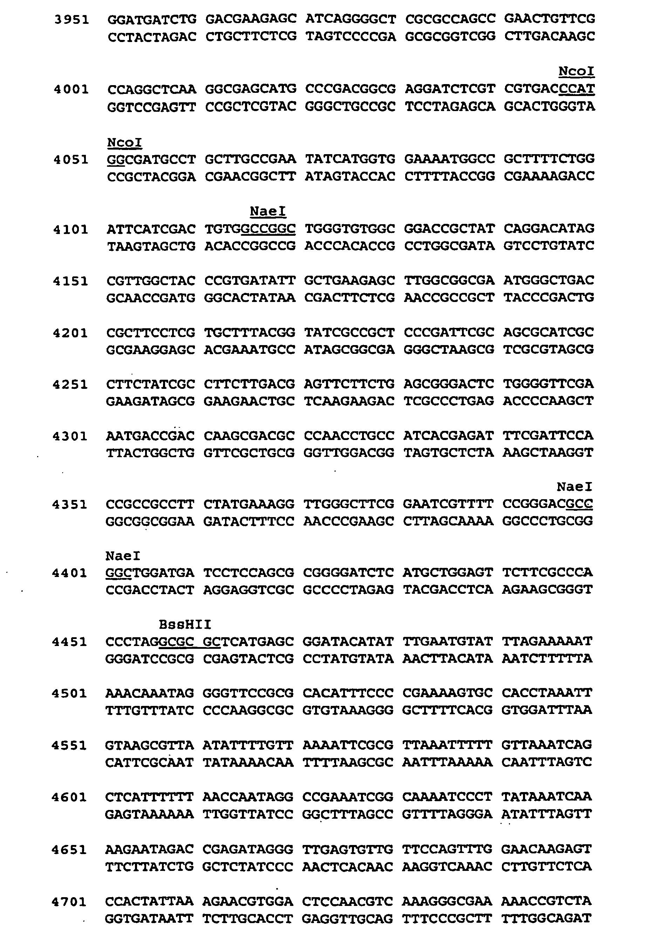

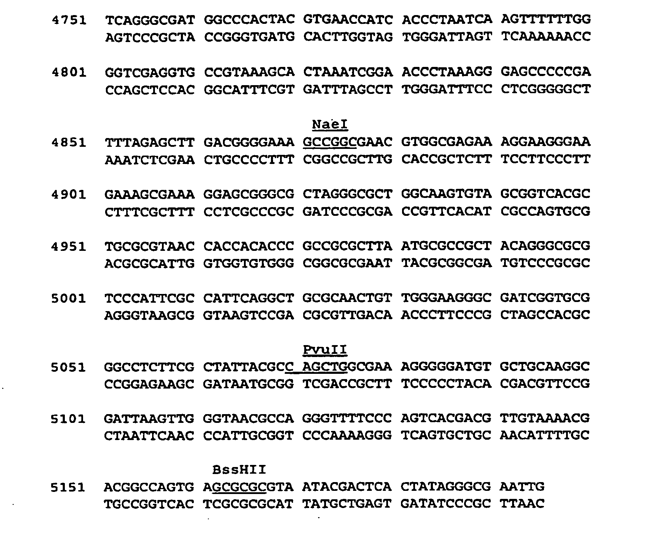

A Del-1 expression system was developed incorporating the cytomegalovirus (CMV) promoter, a 5' synthetic intron, the hDel-1 cDNA, and the 3' polyadenylation signal and untranslated region from the human growth hormone gene (figure 3b). In addition to the expression plasmid encoding human Del-1, an analogous murine Del-1 expression plasmid has been constructed. The mDel-1 plasmid has been utilized in some preclinical murine studies. The Del-1 expression cassette shown in Figure 3b is contained in a plasmid backbone containing the bacterial gene for kanamycin resistance (Figure 3c), which allows for selective growth during plasmid production. Use of other expression backbones including for example alternative promoters, 5' and 3' untranslated regions, polyadenylation signals may be employed and are well known in the art. Figure 12 depicts the nucleotide sequence for human Del-1 while Figure 13 depicts the nucleotide sequence of human Del-1 expression plasmid pDL1680 shown schematically in Figure 3c.

### Example 3: Pharmacology of Del-1 gene medicine

*Del-1 Western:* The protein product from the Del-1 encoding vector was analyzed by performing a SDS-PAGE gel. The lyophilized muscle tissue was homogenized in 2ml tubes containing 2.5mm silica beads with lysis buffer at 10 microliters/mg wet weight, and the non-soluble material was centrifuged out. A NOVEX Tris-Glycine gel was ran with a high molecular weight marker (SIGMA #C3312), 50 nanograms of a peptide standard (PROGENITOR), or 50 micrograms total protein of unknown samples per lane. The gel was transferred to nitrocellulose membrane, and blocked for 1 hour in PBS/0.1 % Tween-20/5% dry milk/4% BSA. The primary antibody was a rat anti-Del-1 monoclonal added at a dilution of 1:500 into blocking buffer over night. After thorough was washing in PBS/0.1% Tween-20, an anti-rat HRP secondary antibody was added in PBS/0.1% Tween-20 at 1:10,000 dilution. The membrane was incubated for 1 hour, then washed thoroughly and incubated in AMERSHAM ECL chemiluminescent reagent for 1-2 minutes, and exposed to X-OMAT AR film.

*IGEN:* The protein product from the formulated Del-1 expression system was quantitated by an ORIGEN IGEN immunoassay specifically developed for Del-1. Briefly, the lyophilized muscle tissue is homogenized in 2 ml tubes containing 2.5 mm silica beads in lysis buffer at 10 microliters/mg wet weight, and non-soluble material is centrifuged out. The assay utilizes avidin coated beads, a biotinylated capture antibody and a ruthenylated detection antibody. All reagents are incubated to allow binding, then sampled through the IGEN instrument, where a magnetic capture system binds the bead complexes, and the unbound fraction is washed away. An electric current is passed through the bound complexes, resulting in the ruthenylated component emitting a signal that is measured by the detector. The dynamic range of this assay is 10 picograms-100 nanograms/mg total protein in the lysate.

*RT-PCR:* The Del-1 and VEGF-165 were analyzed to identify the presence of Del-1 and VEGF-165 RNA by RT-PCR. The procedure for the RT-PCR was to first harvest the muscles from the hindlimb, lyopholyze and homogenize the tissue. Then the tissues were processed for RNA using bead beating and Tel-Test RNA Stat 60. To eliminate any contaminating DNA from the samples a standard Dnase I (Boehringer Mannheim) procedure was performed. RT-PCR was performed using SUPERSCRIPT^{™} ONE STEP^{™} RT-PCR System (GIBCO/BRL). Both cDNA synthesis and PCR are performed in a single tube using gene specific primers. Primers for both DEL-1 and VEGF are designed to span the synthetic intron of the plasmid. The DEL-1 primers yield a 304 bp fragment (DNA contamination yields 421 bp). Primers are sense 5'-TGA CCTCCA TAG AAG ACA CCG GGA C-3' (SEQ ID NO: 4) and antisense 5'-GTG ATG CAA CCT CCA CAA CAC TAG A-3' (SEQ ID NO: 5). The VEGF primers yield a 724 bp fragment (DNA contaminant would yield 841 bp). The sense primer is the same as Del-1 and the antisense is 5'-GGA GGGGTC ACA GGG ATG C-3' (SEQ ID NO: 6). RT reaction and PCR cycling parameters are as follows. RT at 50° C for 30 min; 95°C for 2 min.; PCR cycling at 95°C for 30 sec; 65°C for 40 sec x 35 cycles. Extension at 65°C for 5 min. then to 4°C.

*Immunohistology for Del-1 and VEGF:* The Del-1 and VEGF-165 gene medicines were visualized in the treated muscle samples by immunohistochemical analysis. Immunohistochemical localization for Del-1 was performed on 5µm cryosections which were immunostained using a rat anti-mouse Del-1 antibody diluted 1:500 for 1 hour after blocking with normal rabbit serum. Muscle sections were then rinsed with PBS and incubated for I hour with a biotinylated rabbit anti-rat IgG (Vector Laboratories) at a dilution of 1:400. Muscle sections were subsequently rinsed with PBS and incubated with avidin-HRP (ABC Elite, Vector Laboratories) for 1 hour. After sections were rinsed with PBS, immune complexes were visualized by reaction with DAB (Vector Laboratories). Immunohistochemical localization for VEGF-165 was performed on 3 mm² paraffin sections. The sections were immunostained using a rabbit anti-human VEGF antibody (Intergen Company) diluted 1:200 for 1 hour after blocking with normal goat serum. Muscle sections were rinsed with PBS and incubated for 1 hour with a biotinylated goat anti-rabbit IgG (Vector Laboratories) at a dilution of 1:400. Muscle sections were subsequently rinsed with PBS and incubated with avidin-HRP (ABC Elite, Vector Laboratories) for 1 hour. After sections were rinsed with PBS, immune complexes were visualized by reaction with DAB (Vector Laboratories).

*Quantitive RT-PCR:* The quantification of the RNA from the treated muscles with formulated Del-1 expression system was analyzed by RT-PCR. The total RNA was isolated from muscle samples using RNAzol. Residual plasmid DNA was removed by treating RNA samples with Dnase. The mRNA in the Del-1 samples was quantified using a single step Taqman RT-PCR assay in an ABI-7700 thermal cycler. The 5' and 3' primers that spanned the intron in the 5' UTR of the Del-1 mRNA were used for amplification of the mRNA specific amplicon.

*Capillary Density and Capillary to Muscle Fiber Ratio:* The capillary density was analyzed by a semi-quantitative western blot for CD-31. Briefly, the lyopholized muscle tissue was homogenized in 2 ml tubes containing 2.5 mm silica beads lysis buffer at 10ul/mg wet weight, and non-soluble material was centrifuged out. A Novex Tris-Glycine gel is run with a high molecular weight marker (Sigma #C3312), 50 nanograms Peptide Standard (RDI), or 50 micrograms total protein of unknown samples per lane. The gel was transferred to nitrocellulose membrane, and blocked for 1 hour in PBS/0.1% Tween-20/ 5% dry milk/4% BSA. The primary antibody, goat anti-CD31 (RDI) was added at 1:500 into the blocking buffer, and incubated at room temperature overnight, with shaking. After thorough washing in PBS/0.1% Tween-20, an anti-goat-HRP secondary was added, in PBS/0.1% Tween-20, at 1:10,000. The membrane was incubated for 1 hour, then washed thoroughly and incubated in Amersham ECL chemiluminescent reagent for 1-2 minutes, and exposed to X-OMAT AR film. The film was scanned into the computer and the pixel density of each band was assessed using ImageQuant software. Since it is possible to have gel-to-gel variations, all numbers are reported as a percentage of the CD31 peptide standard run on the same gel.

The capillary to muscle fiber ratio was determined by immunohistochemical localization for CD-31. Samples of muscle tissue that were 5 mm² cryosections were immunostained using a rat anti-mouse CD 31 antibody (PharMingen) diluted 1:1000 for 1 hour after blocking with normal rabbit serum. Muscle sections were rinsed with PBS and incubated for 1 hour with a biotinylated rabbit anti-rat IgG (Vector Laboratories) at a dilution of 1:400. Muscle sections were subsequently rinsed with PBS and incubated with avidin-HRP (ABC Elite, Vector Laboratories) for 1 hour. After sections were rinsed with PBS, immune complexes were visualized by reaction with DAB (Vector Laboratories).

The image analysis for capillary to myocyte ratio was performed using the Optimas image analysis software using a custom macro for counting capillaries and myofibers. Images from cryosections immunostained for CD-31 were analyzed and a ratio of capillaries to myofibers was established.

*Angiogra Assessment:* Angiography was used to evaluate the collateral arteries that had developed during the rabbit hindlimb ischemic model experiment. The right femoral artery was catheterized and injected with 4 mls of contrast agent (VISIOPAQUE™). Serial images of both hindlimbs were recorded. Quantitative angiographic analysis of the collateral vessel development was performed by a blinded observer directly counting the number of vessels crossing two perpendicular planes measured from anatomical landmarks on the femur and then averaged. The image used for the analysis was taken at 130 frames on the cine loop taken from the camera using DIACOM VIEW ™ software. This analysis was performed three separate times and the average of the observations was recorded.

*Treadmill:* A treadmill stress test was performed to analyze the physiological changes with the administration of the Del-1, VEGF-165, non-coding plasmids along with a sham operative control group. The animals were placed on the treadmill at a seven degrees angle for five minutes at five meters/minutes to acclimate to the treadmill. After the initial five minutes, the treadmill speed is increased to ten meters/minutes and the clock started. The speed is increased every two minutes until signs of fatigue are noticed. The signs of fatigue are a change in gait, unable to run to the front of the treadmill and spending more than five seconds on the shocker grid. When any combination of these signs is observed the animal is removed and the time is stopped.

*Statistical Analysis:* All results are expressed as means + SEM. Statistical significance was evaluated using a one-way ANOVA with Duncan as the post-hoc analysis. A P value< 0.05 was interpreted to denote statistical significance.

*Animal Models:* The preclinical pharmacology of the formulated Del-1 expression system has been evaluated in mouse and rabbit animal models. Intramuscular injection of formulated Del-1 plasmid has been shown to increase capillary density in normoxic skeletal muscle and to increase collateral vessel formation and exercise tolerance in animal models of limb ischemia. The biological effects of the formulated Del-1 plasmid are dose dependent. No evidence of toxicity or gross pathology related to the expressed Del-1 has been observed in animal models.

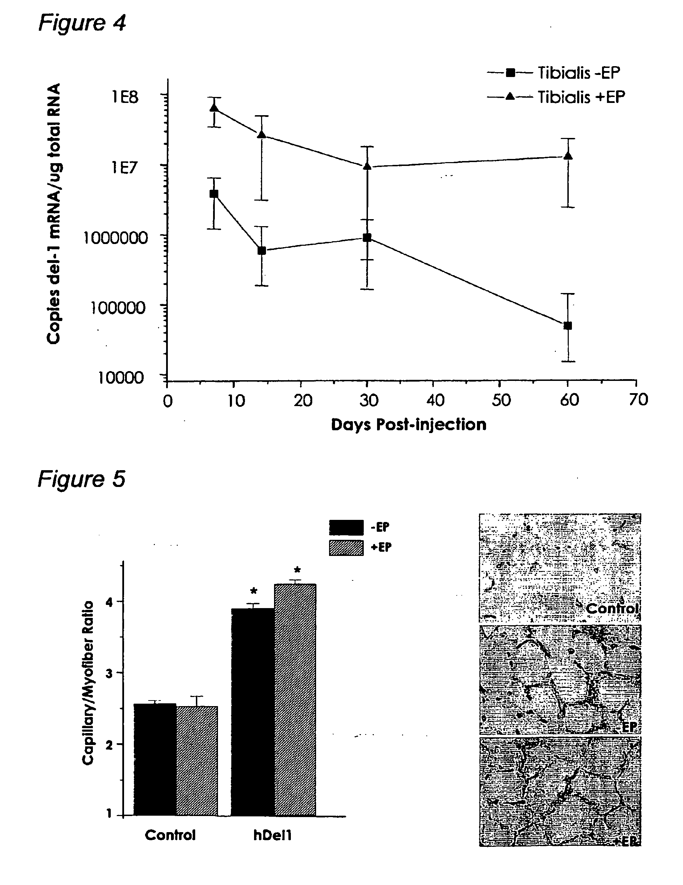

The level and duration of mDel-1 expression in tibialis anterior muscles of mice following injection of formulated mDel-1 plasmid with and without electroporation are presented in figure 4. Ten micrograms formulated Del-1 plasmid were injected into the tibialis anterior muscles of CD-1 mice. Injected muscles were harvested at 7, 14, 30 and 60-day post injection and assayed for mDel-1 mRNA by qrtPCR. Data points shown represent the mean +/- SEM for n=5/group/time point. Results from this experiment indicate that expression of mDel-1 decreased at the rate of approximately one log per month when administered without electroporation. Administration of mDel-1 encoding nucleic acids in conjunction with electroporation resulted in an approximate two log increase in the level of Del-1 mRNA, and furthermore, appeared to increase the persistence of mDel-1 expression.

The effect of hDel-1 gene expression in normoxic tibialis anterior muscle of mice 7-day post injection with and without electroporation was determined as is shown in Figure 5. Mice were injected IM into the tibialis anterior with formulated Del-1 or control plasmid followed by use of electroporation to further enhance plasmid uptake (+EP) in half of the injected muscles. Panel A: Results in the bar graph depict capillary density at 7 days post-treatment determined by computer image analysis of CD31 immunostaining. Data show the mean +/- SEM for n=3/group. An asterisk indicates that the groups are different from control (p<0.01). Panel B: Photographs show representative CD-31 immunostaining in muscle cross-sections. A single 10 microgram dose of hDel-1 plasmid DNA increased capillary:myofiber ratio by approximately 60 % (p<0.01). Increasing the level of hDel-1 expression through the use of electroporation did not further increase capillary:myofiber ratio. Although not shown, the effects of human and murine Del-1 in this model were equivalent.

The immunohistological analysis showed that the formulated Del-1 plasmid caused a very minimal inflammatory effect, whereas the VEGF-165 formulated plasmid resulted in a massive infiltration of inflammatory cells. The inflammation seen with formulated VEGF-165 expression system agrees with other studies showing the increase in inflammatory cells even seeing the extreme of hemangiomas when VEGF-165 is expressed for long amounts of time (Springer M, et al. Molecular Cell. 1998;2:549-558; Ozawa C, et al. Annu. Rev. Pharmacol. Toxicol. 2000; 40:295-317).

### Example 4: Dose response relationship

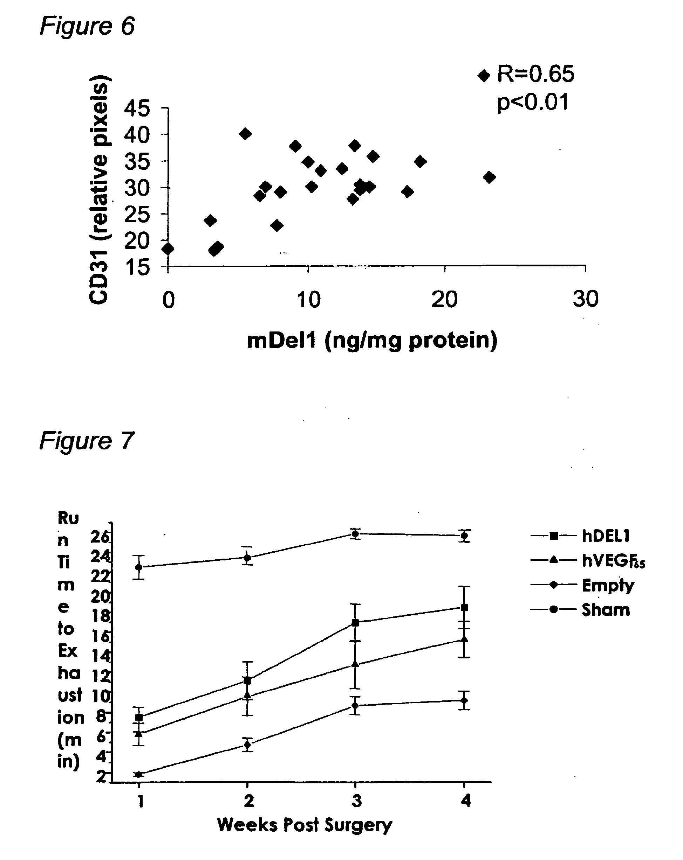

To investigate the relationship between expressed Del-1 protein and increased expression of the capillary endothelial surface antigen CD31 experiment was performed to quantify the concentration of expressed mDel-1 and CD31 within the injected muscle. Mice were injected instramuscularly with either 10 micrograms non-coding plasmid, 10 micrograms Del-1 plasmid, 20 micrograms Del-1 plasmid, or 30 micrograms Del-1 plasmid administered in conjunction with electroporation. Muscles were harvested 7 days post injection and assayed for mDel-1 by sandwich immunoassay and for CD31 by Western blot followed by densitometry. Results from this experiment are shown in Figure 6. Expression of mDel-1 was strongly correlated with expression of the capillary endothelial surface antigen CD31 (R=0.65, p<0.01). The approximate EC₅₀ for the Del-1 concentration dependent increase in CD31 was approximately 5 ng/g wet muscle.

### Example 5: Effects of formulated Del-1 plasmid in murine hind limb ischemia

In addition to studies in normoxic mouse hindlimb, the effects of formulated Del-1 plasmid have been investigated in a murine hind limb ischemia model. CD-1 mice (26-31gm) were obtained from Charles River (Houston). Briefly, after sedation with ketamine (7.4 mg/gm), xylazine (0.4 mg/gm) and acepromozine (0.08mg/gm), the femoral artery was ligated in both legs. A longitudinal incision was made on the medial side of the thigh inferiorly from the inguinal ligament down to a point proximal of the patella. With this incision, the femoral was ligated at its proximal origin where it branched from the iliac to the point distally where it bifurcated into the popliteal and sapheneous arteries.

Immediately after ligation of the femoral artery, formulated hDel-1 plasmid, formulated hVEGF plasmid or control (non-coding) plasmid was injected intrasmuscularly (i.m.) with a 28 gauge 1/2" needle in five different places into the thigh and lower limb (70 micrograms total dose/hindlimb divided among the tibialis anterior (10 micrograms), gastrocnemius (20 micrograms), and quadriceps (40 micrograms) muscles and administered immediately following surgery). Each injection consisted of 0.1 ml of a poloxamer formulation at a concentration of 1mg/ml. Revascularization was assessed by the physiological endpoint of a treadmill stress test and by CD-31 semi-quantitative western blot analysis. Exercise tolerance was determined at weekly intervals through four weeks post surgery. Formulated VEGF plasmid was included for comparison since numerous studies have indicated that overexpression of VEGF can lead to increased revascularization in ischemic tissue.

Data shown in Figure 7 represent the mean +/- SEM for n=7-8 animals/group. Different superscripts associated with different groups indicated that the exercise times for the groups are different (p<0.05). Results from this study presented in Figure 7 show that both Del-1 and VEGF gene medicines increased exercise tolerance versus control (p<0.05). An evaluation of mouse skeletal muscle treated with formulated Del-1 plasmid over a six month time period by qRT-PCR showed that, after six months, the formulated Del-1 plasmid has not significantly decreased in expression.

### Example 6: Effects of formulated Del-1 plasmid in the rabbit femoral artery excision ischemia model

The most common animal model employed for investigation of angiogenic factors in hindlimb ischemia is the rabbit femoral artery excision model. A study of similar design to that described above in mice was conducted in New Zealand rabbits. The first model used 14 male New Zealand Rabbits (4 kg) to create the rabbit hindlimb ischemic model. Briefly, after sedation with ketamine (20mg/kg), xylazine (5mg/kg) and acepromozine (2.5mg/kg), the femoral artery was resected from the left leg. A longitudinal incision was made on the medial side of the thigh inferiorly from the inguinal ligament down to a point proximal of the patella. With this incision, the femoral artery was dissected free and the connecting arterial branches were dissected free and ligated. The femoral artery was now excised from its proximal origin where it branched from the external iliac to the point distally where it bifurcated into the popliteal and sapheneous arteries.

For the rabbit hindlimb ischemia model, the non-viral formulated DNA encoding Del-1, VEGF -165, or a non-coding plasmid for a control was injected instramuscularly with a 28 gauge 1/2" needle in ten different places into the medial portion of the thigh of the rabbit three days post surgery. The concentration of the DNA used was 1mg/ml formulated with poloxamer 188 with each injection consisting of 0.5ml for a total of 5 mg of DNA.

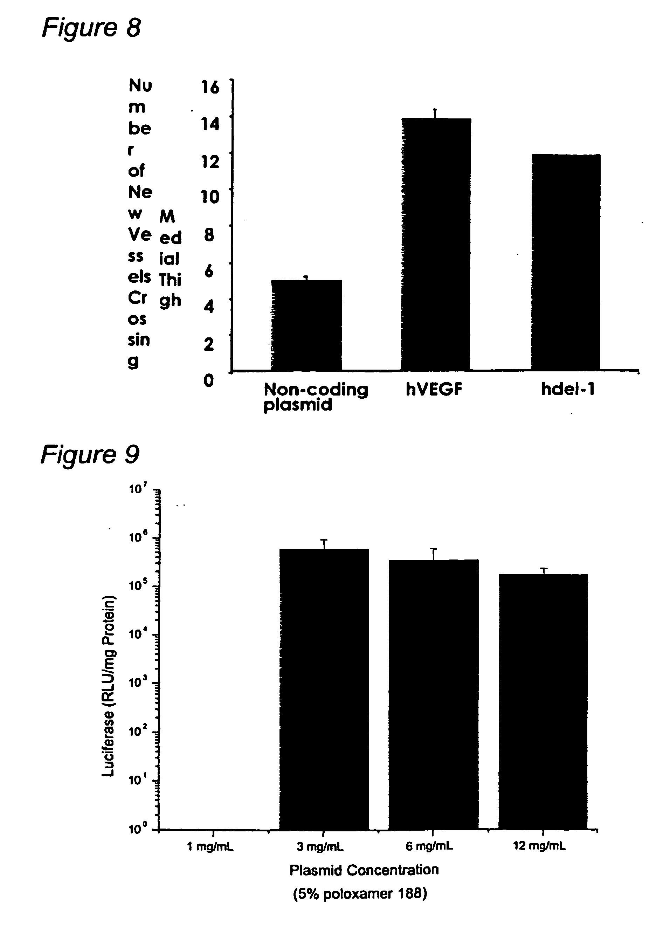

Angiography was performed immediately after surgery and again at one month. Revascularization of the ischemic limb was evaluated 30 days post-treatment by CD-31 semi-quantitative western blot analysis and angiography analysis. Results for the number of new collateral vessels crossing over the mid thigh region are shown in Figure 8. Numbers of animals/group were n=3 (control), n=5 (Del-1), and n=6 (VEGF). Bars represent the mean +/- SEM for the number of new collateral vessels crossing over the mid thigh. Bars with different superscripts are different (p<0.01). Both Del-1 and VEGF elicited a more than two-fold increase in collateral vessel development over the one month course of the experiment (p<0.05).

### Example 7: Optimization of the formulation and delivery of Del-1 plasmid to myocardium

Experiments to identify and optimize the formulation and delivery of Del-1 plasmid DNA to myocardium were conducted. Results suggest that maximal delivery efficiency to the left ventricle is attained with a polymer formulation administered via retrograde IV infusion. Eight pigs were dosed with formulated plasmid via this route (4 with poloxamer, 4 with cationic lipids) and the procedure was well tolerated. However, at 7 days gross pathology was noted in the myocardium of pigs dosed with cationic lipid formulations. The delivery of contrast media or dye via that procedure resulted in localized extravasation into the parenchyma of the left ventricle. Expression of Del-1 mRNA was highest in pigs dosed with the poloxamer formulation. Results from experiments conducted in a murine model to assess different formulations for direct intramyocardial injection suggest that either formulation with polyglutamate or increasing the concentration of plasmid from 1 mg/ml to 3 mg/ml appear to enhance plasmid delivery to myocardium when administered by needle injection.

### Example 8: Percutaneous Delivery the Myocardium

Potential percutaneous delivery modalities were tested in pigs and compared based on safety, technical feasibility, and plasmid delivery efficiency. Gene expression and tolerability data for these experiments are summarized in Table 1. For all percutaneous delivery experiments conducted major organs have been sampled to assess biodistribution of the plasmid at harvest (usually 7 day post-administration). Results (not shown) reveal that the plasmid is primarily localized to the heart regardless of the route of administration or formulation.

### Delivery via needle injection catheter

Between two experiments a total of N=6 pigs were dosed with Del-1 plasmid DNA formulated in either poloxamer 188 (n=4) or saline (n=2) and administered via helical needle injection catheters provided by BioCardia, Inc. The delivery procedure was well tolerated in all animals and no remarkable findings of gross pathology were made at the time of harvest. Expression data shown on Table I indicated low level Del-1 expression localized within the injected left ventricle using IM needle catheter delivery in contrast to the high levels obtained with retrograde venous delivery.

**Table 1**

| | Del-1 mRNA in LV¹ | Percent positive sections | Del-1 mRNA in positive sections ² |
|---|---|---|---|
| IM Catheter | 23,589 | 20 | 120,184 |
| (10 sites) | 6,136 | 16 | 18,949 |
| | | | |
| Retro IV | 114,020 | 28 | 405,075 |
| | 806,555 | 41 | 2,177,932 |

| | | | |
|---|---|---|---|
| ¹Copies Del-1 mRNA/microgram total RNA in all sections analyzed, LOQ = 500 copies/microgram (500,000-1,000,000 typical level achieved in murine limb muscle) ²Copies Del-1 mRNA/microgram total RNA (average of positive sections) | | | |

Delivery of a formulated DNA to the myocardium with a needle injection catheter is most analogous to intramuscular injection using a needle and syringe. Results from mouse studies shown in proceeding sections of this report suggest that intramuscular injection may not be as efficient for introducing gene medicines into myocardium as it is for skeletal muscle.

### Delivery to the pericardial space

Delivery to the pericardial space was accomplished via use of a trocar device (PERDUCER^{™}) from Comedicus, Inc. A positively charged cationic lipid formulation was tested via this route. The delivery procedure appeared to be well-tolerated. Low level expression of Del-1 mRNA (<500 copies/micrograms total RNA) was detected in myocardial tissue.

### Delivery to the left ventricle via retrograde IV infusion

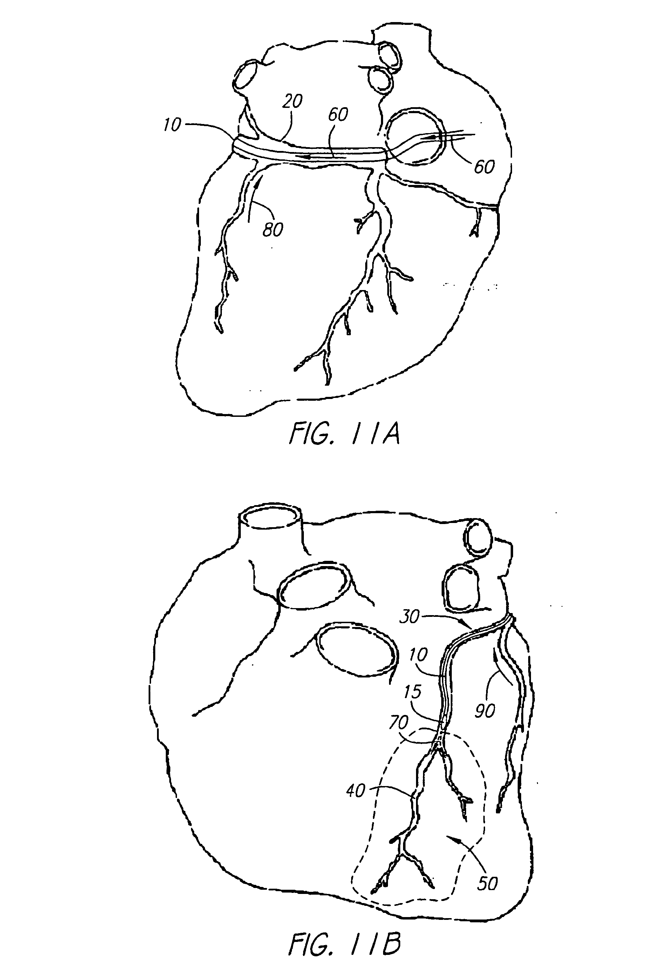

Retrograde IV ("rIV") delivery to the left ventricle was accomplished via placement of a 7F balloon catheter (10, shown in Figures 11A and 11B) through the coronary sinus (20, Figure 11A), the great cardiac vein (30, Figure 11B), and into the mid region of the anterior intraventricular vein (40). After the inflation of the balloon (15) to occlude venous outflow, injection of 10 ml of formulated plasmid was performed either by maximum hand pressure or at a controlled rate; the rate being either a volume/time rate such as 1 ml/second, or at a predetermined pressure. Delivery of contrast media or dye via this procedure resulted in localized extravasation (50) of the media into the parenchyma of the left ventricle. The arrows (60 and 70) on Figures 11A and 11B show the direction of flow for the formulated plasmid through and from the catheter (10). The arrows (80 and 90) on Figures 11A and 11B show the direction of blood flow in the vein.

Following delivery of the formulated plasmid the inflated balloon (15) was left in place for several minutes (2 - 10 minutes in pigs depending on the experiment) to increase residence time of the formulation within the tissue. Eight pigs were dosed with formulated plasmid via this route (n=4 with poloxamer formulation, n=4 with cationic lipid formulations). The delivery procedure was well-tolerated in all animals. However, upon harvest 7 days post administration significant gross pathology was noted in the myocardium of pigs dosed with cationic lipid formulations. Pathology appeared to be more severe in pigs dosed with the 1:3 (-/+) formulation than with the 1:0.5 (-/+) formulation.

Expression of Del-1 mRNA was highest in pigs dosed with the poloxamer 188 formulation and decreased significantly with higher concentrations of cationic lipid as shown in Table 2. Of the delivery modalities and formulations tested only the poloxamer formulation administered via retrograde IV infusion yielded levels of expression that were comparable to those typically achieved in murine limb muscle following IM injection. Both deliveries via intramyocardial injection and via retrograde IV infusion of a poloxamer formulation appear to be well-tolerated.

**Table 2. Summary of data from percutaneous myocardial delivery studies conducted to date.**

| | **Formulation** | **Technical Success¹** | **Del-1 mRNA²** | **Gross pathology** |
|---|---|---|---|---|
| IM catheter | Poloxamer 188 5% | 6/6 | Detectable³ (n=4) | Negative |
| Pericardial | Cationic lipid (1:3, -/+) | 1/3 | <LOQ (n=3) | Mild |
| Retrograde IV | Cationic lipid (1:3, -/+) | 2/2 | <LOQ (n=2) | Moderate/severe |
| Retrograde IV | Cationic lipid (1:0.5, -/+) | 2/2 | 3997 (n=2) | Mild/moderate |
| Retrograde IV | Poloxamer 188 5% | 4/4 | 397279 (n=2) | Negative |

| | | | | |
|---|---|---|---|---|
| ¹Technical success is defined as the proportion of delivery procedures that were accomplished without significant problems. ²Copies Del-1 mRNA/micrograms total RNA, LOQ=500 copies (500,000-1,000,000 typical level achieved in murine limb muscle). ³Partial degradation of RNA during shipping precluded quantification of Del-1 mRNA in these samples. | | | | |

### Example 9: Direct IM injection of formulated plasmid in mouse heart

Because direct intramyocardial injection can be performed in rodents, experiments were conducted to assess the utility of various polymer formulations for delivery of gene medicines to myocardium. Observations made in these experiments are considered in the selection of formulations/formulation parameters for testing in larger animals with a needle injection catheter. All experiments described in the section below were conducted in mice using the luciferase reporter plasmid pLC0888. In brief, mice are anesthetized with pentobarbital, intubated, and then the chest opened via an incision along the midline of the sternum. The heart, beating at 500-600 beats/min, was gently elevated in the chest cavity using a sterile swab and formulated plasmid injected with an insulin syringe. Survival following the injection procedure is generally greater than 80 % unless otherwise affected by toxicity of the formulation. For comparative purposes it should be noted that the level of luciferase expression observed in mouse skeletal muscle 7d following injection of pLC1088 formulated in 5% poloxamer 188 is approximately 10⁷ RLU/mg protein.

The objective of this experiment was to determine whether increasing the concentration of plasmid in a poloxamer 188 formulation would increase delivery of pLC0888. Nonetheless, results from this experiment presented below in figure 9 suggest that increasing the concentration of plasmid in the 5% poloxamer 188 formulation from 1 mg/ml to 3 mg ml increases delivery efficiency to cells in the myocardium. Data shown represent the mean +/- SD for n=3/group. CD-1 mice were injected with the 1, 3, and 12 mg/ml formulations. C57 BL6 mice were injected with the 6 mg/ml formulation.

Further experiments have indicated that direct IM injection of a 1mg/ml plasmid, 5% poloxamer formulation does result in measurable levels of luciferase expression in the injected heart.

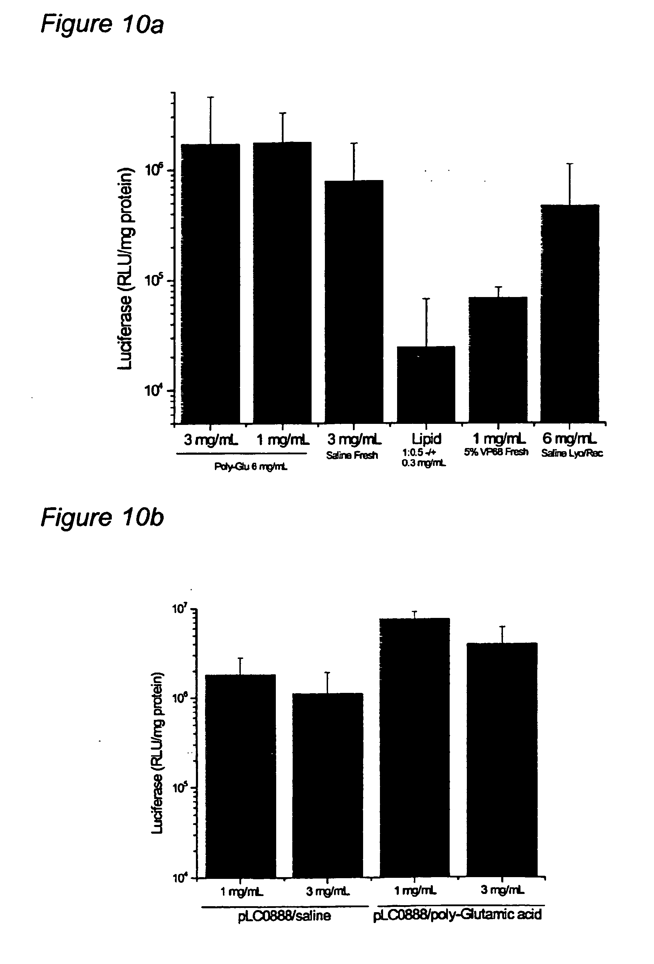

A comparison of various formulations for delivery of plasmid to myocardium via intramyocardial injection was conducted. Results shown in Figure 10a indicate that formulation of plasmid in poly glutamic acid yields more efficient plasmid delivery than the other formulations tested. An additional study the results of which are shown in Figure 10b has confirmed this result versus formulation in saline. Data represent the mean +/- SD for n=3/group (panel A), n=4-5/group (panel B).

### Example 10: Non-condensing polymer formulated plasmid

In one embodiment of the present invention, the compound in which the DNA is formulated can be shown to interact with the DNA by one or more of the following methods: FTIR; ITC; DELSA; and fluorescence quenching. In certain circumstances, the compound may be shown to provide protection against nucleases *in vitro.* Preferred compounds do not result in condensation of the DNA but do facilitate dispersion and delivery to solid tissues (e.g., muscle, tumors) and effect an increased extent and levels of expression. As shown in Figure 12, preferred compounds effect an increased reproducibility of expression.

### Example 11: Formulated Del-1 Expression Plasmid Pharmaceutical Product

In one embodiment of the present invention, the facilitating agent is Poloxamer 188 having the following chemical composition:
HO(CH₂CH₂O)₈₀(CH(CH₃)CH₂O)₂₇(CH₂CH₂O)₈₀H.

In one embodiment the del-1 drug product includes:

| **Component** | **Drug Product Composition (mg/vial)** |
|---|---|
| Drug Substance (pDL1680) | 5.0 |
| Facilitating Agent (Poloxamer 188) | 250 |
| Excipients: (TRIS) | 0.45 |
| (TRIS-HCl) | 0.70 |

In one embodiment the formulated DNA is prepared by aseptically mixing equal volumes of the plasmid DNA, pDL1680, and the poloxamer 188 as follows:

After filtration, the composition or mixture may be lyophilized and stored. When needed for use, the lyophilized composition can be rehydrated in normal saline solution.

### Example 12: Formulated Del-1 Expression Plasmid Delivered with Electroporation

The term "pulse voltage device", or "pulse voltage injection device" as used herein relates to an apparatus that is capable of causing or causes uptake of nucleic acid molecules into the cells of an organism by emitting a localized pulse of electricity to the cells, thereby causing the cell membrane to destabilize and result in the formation of passageways or pores in the cell membrane. It is understood that conventional devices of this type are calibrated to allow one of ordinary skill in the art to select and/or adjust the desired voltage amplitude and/or the duration of pulsed voltage and therefore it is expected that future devices that perform this function will also be calibrated in the same manner. The type of injection device is not considered a limiting aspect of the present invention. The primary importance of a pulse voltage device is, in fact, the capability of the device to facilitate delivery of compositions of the invention into the cells of an organism. The pulse voltage injection device can include, for example, an electroporetic apparatus as described in U.S. Patent 5,439,440, U.S. Patent 5,704,908 or U.S. Patent 5,702,384 or as published in PCT WO 96/12520, PCT WO 96/12006, PCT WO 95/19805, and PCT WO 97/07826, all of which are incorporated herein by reference in their entirety.

The term "apparatus" as used herein relates to the set of components that upon combination allow the delivery of compositions of the invention into the cells of an organism by pulse voltage delivery methods. The apparatus of the invention can be a combination of a syringe or syringes, various combinations of electrodes, devices that are useful for target selection by means such as optical fibers and video monitoring, and a generator for producing voltage pulses which can be calibrated for various voltage amplitudes, durations and cycles. The syringe can be of a variety of sizes and can be selected to inject compositions of the invention at different delivery depths such as to the skin of an organism such as a mammal, or through the skin.

In one embodiment, administration of vector (plasmid) and use of formulations for delivery are by pulse voltage delivery to cells in combination with needle or needle free injection, or by direct applied pulse voltage wherein the electroporation device electrodes are pressed directly against the targeted tissue or cells, such as for example epidermal cells, and the vector is applied topically before or after pulse application and delivered through and or to the cells.

The route of administration of any selected vector construct will depend on the particular use for the expression vectors. In general, a specific formulation for each vector construct used will focus on vector delivery with regard to the particular targeted tissue, the pulse voltage delivery parameters, followed by demonstration of efficacy. Delivery studies will include uptake assays to evaluate cellular uptake of the vectors and expression of the DNA of choice. Such assays will also determine the localization of the target DNA after uptake, and establishing the requirements for maintenance of steady-state concentrations of expressed protein. Efficacy and cytotoxicity can then be tested. Toxicity will not only include cell viability but also cell function.

Muscle cells have the unique ability to take up DNA from the extracellular space after simple injection of DNA particles as a solution, suspension, or colloid into the muscle. Expression of DNA by this method can be sustained for several months.

Studies were conducted to determine the potential of Del-1, when delivered IM as a non-viral gene medicine, to increase vascular density in hindlimb muscle of mice. Briefly, 10 micrograms of PINC^{™} polymer-formulated mDel-1, hDel-1 or hVEGF₁₆₅ plasmid (10 micrograms plasmid dose) were injected into the tibialis anterior muscles of male CD-1 mice. In some instances, electroporation was utilized to further enhance the transfection efficiency of myofibers over that achieved with formulated plasmid. Injected muscles were harvested 7 days post-treatment, snap frozen, cryo-sectioned, and immunohistochemical analyses for the capillary endothelial surface antigen CD 31, Del-1, and VEGF₁₆₅ were performed. Images were analyzed using the OPTMAS image analysis software equipped with a custom macro to calculate the capillary:myocyte ratio in skeletal muscle. Electroporation increased transfection efficiency and transgene expression by as much as two logs. Electroporation of untreated muscles resulted in little or no detectable pathology and did not affect capillary:myofiber ratio (2.56 nonelectroporation versus 2.52 electroporated). Injection of either formulated del-1 or VEGF₁₆₅ expression plasmids elicited significant increases in capillary:myofiber ratio (p<0.05) that ranged between 30 and 50 percent above control values. Enhanced transgene expression resulting from electroporation further enhanced capillary:myocyte ratio by as much as 70% versus control although the effect of electroporation was not significant in all cases. Increased immunostaining for CD 31 was observed in close proximity to areas of transgene expression. The pattern of CD 31 immunostaining was similar in muscles injected with either formulated Del-1 or VEGF₁₆₅ expression plasmids. However, pharmacological levels of VEGF₁₆₅ achieved through the use of electroporation were associated with vessel clusters and severe localized edema, which were not observed in other treatment groups. These results suggest that non-viral formulated Del-1 expression system may be useful in stimulating the re-vascularization of ischemic tissue and point to the utility of electroporation as a tool for investigating the dose response and therapeutic index of non-viral gene medicines.

### Example 13: Comparison of expression levels measured after delivery of plasmid formulated with either saline or poloxamer to pig myocardium

Studies were undertaken to compare expression levels measured after delivery of plasmid (1 mg/mL) formulated in saline or in 5% poloxamer. pDL1680 (1 mg/ml) with 5% poloxamer 188 in isotonic saline was compared with pDL1680 (1mg/ml) in isotonic saline. SPF Yorkshire pigs (approximately 50 kg) were used in the studies.

Test article (10 ml, 10 mg plasmid dose) was delivered into the mid region of the AIV (or other vein if AIV could not be accessed) by rIV infusion using a balloon catheter. The catheter was positioned in the target vein under fluoroscopic guidance with radiographic contrast medium used as needed. The delivery site was assessed by administering contrast medium to determine the region of distribution (i.e., blush), and to ensure correction positioning of the catheter to minimize collateral drainage. Following positioning of the catheter a 10 ml bolus of formulated Del-1 plasmid was delivered under either maximal hand pressure or over a period of approximately 10 seconds or less. Results indicated that delivery under maximal hand pressure increased transfection of the myocardium compared with administration under controlled pressure.

The target region of myocardium identified as the region of most intense blush when contrast medium was administered immediately prior to administration of test article was harvested (total area approximately 4.5 x 4.5 cm) and sectioned into approximately 1 x 1 cm cubes. These tissue samples, as well as samples form more distal regions of myocardium and samples of lung, liver, kidney, and spleen were assayed for hDel-1 transgene mRNA by quantitative rtPCR ("qrtPCR"). In addition, immunohistochemistry for hDel-1 protein was performed on samples of myocardium taken from the target region of delivery.

The delivery procedure was well-tolerated with no major in-life events or gross pathology associated with the myocardium noted. Minor bruising was noted at the site of balloon inflation in some pigs. Figure 15 shows expression of hDel-1 mRNA within the myocardium of pigs treated by rIV delivery with either pDL1680 formulated in saline or pDL1680 formulated with 5 % poloxamer 188. Data points represent the mean hDel-1 mRNA level within the 4.5 x 4.5 cm target region for delivery. Solid circles show results with maximum hand pressure while open circles show results with timed administration. Pooled treatment group means are shown as horizontal lines in the figure. Results for hDel-1 mRNA shown in Figure 15 indicate that formulation with 5 % poloxamer 188 yielded approximately 6x higher levels of hDel-1 mRNA within the targeted region of myocardium versus formulation in saline (p=0.036) regardless of whether maximum hand pressure or timed administration was performed. Expression of the hDel-1 transgene in more distal regions of the myocardium and distal organs was absent or negligible and unaffected by formulation.

### Example 14: Use of Del-1 and VEGF in combination for angiogenesis

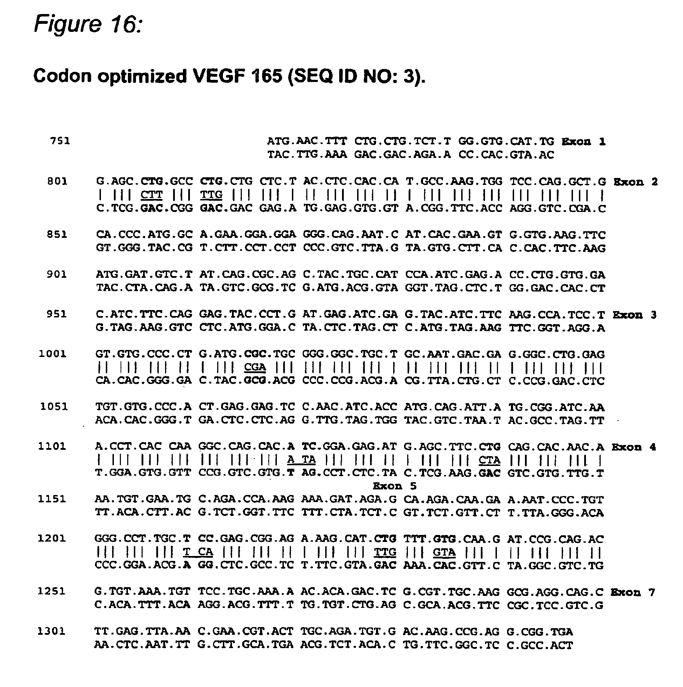

The cDNA encoding VEGF 165 was chemically synthesized and cloned into a mammalian expression plasmid having a (CMV) promoter, a 5' synthetic intron, the hDel-1 cDNA, and the 3' polyadenylation signal and untranslated region from the human growth hormone gene as with the Del-1 expression plasmid shown in Figure 3b. The coding sequence of VEGF used is shown on Figure 16. The codon usage of VEGF 165 was analyzed respective to the known preferred codons found in highly expressed human proteins. Eight codons of the native VEGF 165 sequence that represent rarely used codons were altered to conform with those codons used in highly expressed human proteins. The modified codons are shown on Figure 16 where the native codon is depicted between the strands.

### In Vitro assay of Del-1 and VEGF combination in tubule formation

Using Primary Endothelial Cells from Human Umbilical Veins (HUVEC cells) a 3D-collagen gel was constructed in which Del-1, VEGF, or Del-1/VEGF was added. Cells were photographed at 2 days to determine the proficiency of Del-1, VEGF, or the combination in inducing tubule formation. The combination group of Del-1 and VEGF showed superior tubule formation to control, Del-1, or VEGF alone.

### In Vivo assay of Del-1 and VEGF combination in capillary development

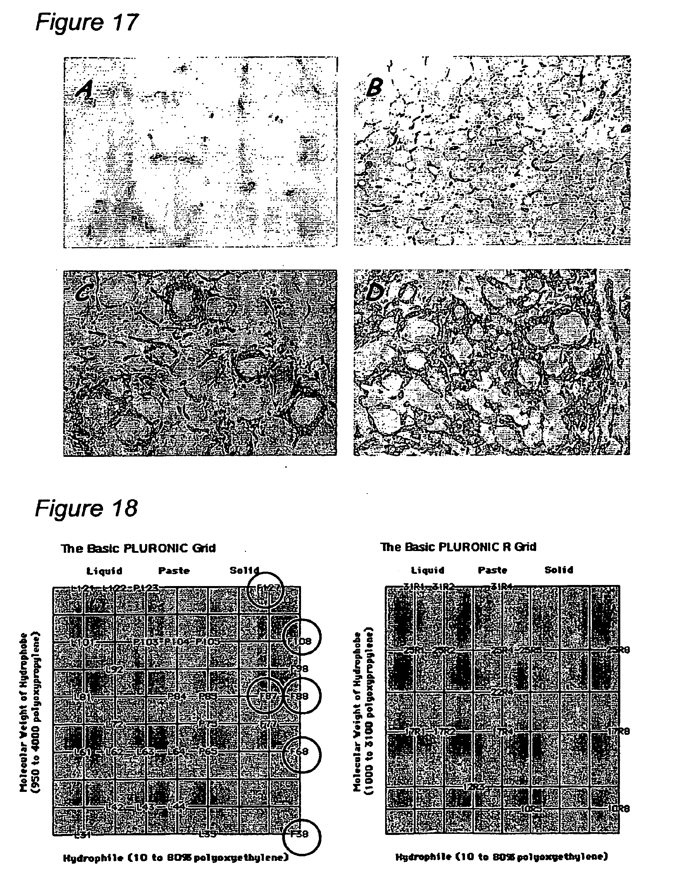

Ten total micrograms of 5% poloxamer 188 formulated plasmid DNA encoding Del-1 (10 micrograms), VEGF (10 micrograms), Del-1 and VEGF (at 5 micrograms per plasmid), or an empty vector control were injected into the anterior tibialis of male CD-1 mice. Immediately following injection of the formulated plasmid DNA, electroporation was performed. Approximately, two minutes after injection, an electric field was applied in the form of 2 square wave pulses (one per second) of 25 millisecond ("ms") each and 375 V/cm delivered by an Electro Square Porator (T820, Genetronics, San Diego, CA). The clamp electrodes consist of 2 stainless steel parallel plate calipers (1.5 cm²) that are placed in contact with the skin so that the leg is held in a semi-extended position throughout pulse administration. Typically the leg of the mouse was positioned between the two plates, which were compressed together until snug with a 3-4 mm separation distance between the plates. Transgene mRNA expression was measured using rt-PCR at 7 and 28 days, and capillary to myocyte ("C/M") ratios were calculated by immunohistochemistry on anti-CD31 stained tissue. Transgene mRNA expression showed elevated expression at 7 days, which decreased by several logs at 28 days. As shown in Figure 17, C/M ratios for Del-1 and VEGF alone were 1.5 to 2-fold better than empty vector, confirming earlier experiments. Suboptimal doses of Del-1 and VEGF in combination yielded a remarkable increase in capillary endothelial cell density in comparison with optimal doses (twice the dose of either) of the genes alone. In the Del-1/VEGF combination, resulting CD-31 positive endothelial cells were too numerous to count. The combination of Del-1 and VEGF was also shown experimentally to increase exercise tolerance over control plasmid or VEGF alone.

Although the combination of Del-1 and VEGF in this example was formulated using two separate plasmids, one skilled in the art would understand that Del-1 and VEGF can also be expressed from a single plasmid carrying two transcription expression units or from a single transcription expression unit having an internal ribosomal entry site.

### Example 15: Poloxamer formulations for gene delivery by retrograde delivery

In the nomenclature of poloxamers, the non-proprietary name "poloxamer" is followed by a number, the first two digits of which, when multiplied by 100, equals the approximate molecular weight ("mw") of the polyoxypropylene ("POP") and the third digit, when multiplied by 10 equals the approximate % by weight of the polyoxyethylene ("POE"). Thus, poloxamer 188 would have an average POP mw of approximately 1800 and an average POE % of 80%. Calculated according to the poloxamer nomenclature for poloxamer 188 (a.k.a. F68) the average number of POP groups are derived as follows: 1800 + 58 *(mw of C₃H₆O)* = 31 POP units. The total mw = 1800 + (20/100) = 9000. The average number of POE are derived as follows: (total approximate mw - mw POP) + 44 *(mw of C₂H₄O)* is thus (9000-1800) = 7200 + 44 = 163. Therefore the formula for poloxamer 188 (a.k.a. F68): HO-(C₂H₄O)₈₂-(C₃H₆O)₃₁-(C₂H₄O)₈₂-H.

Alternatively, from the formula HO-(C₂H₄O)ₓ-(C₃H₆O)_{y}-(C₂H₄O)ₓ-H, the average molecular weight, the percentage of POE, and the numbers of POE and POP units can be otherwise derived depending on the variable known. Thus if the total mw and % POE is known the formula can be derived as follows:
Average number of POE groups are derived as follows: (total approximate mw - 18 (the mw of the terminal hydroxy and hydrogen groups)) x wt% POE) = mw POE + 44 = number of POE groups, therefore for F68; ((8400-18) x 80%) = 6705.6 + 44 = 152.4 (+ 2 = 76)
Average number of POP groups can be derived as follows: ((total approximate mw - 18) - mw POE) = mw POP + 58. Therefore for F68: ((8400-18) - 6705.6 = 1676.4 + 58 = 30. The formula for poloxamer 188, a.k.a. F68, would thus be: HO-(C₂H₄O)₇₆-(C₃H₆O)₃₀- (C₂H₄O)₇₆-H

In the BASF nomenclature, a letter describing the physical form of the poloxamer is followed by a first number arbitrarily representing the molecular weight of the POP step-wise up the y axis of the poloxamer grid and the second number representing the % POE. PLURONIC® F68 is the BASF trademark for poloxamer 188. BASF gives 8400 as the average mw for F68 but states an average mw of 8600 for F68NF grade and gives values of POE = 80 (x 2), and POP = 27: therefore the POP mw = 1566, POE % = 81.6% with the resulting formula: HO-(C₂H₄O)₈₀-(C₃H₆O)₂₇- (C₂H₄O)₈₀-H which would have a resulting mw of 18 + 7040 +1566 = 8624

Because in actual practice, poloxamers are typically synthesized according to a process in which a hydrophobe of the desired molecular weight is generated by the controlled addition of propylene oxide to the two hydroxyl groups of propylene glycol followed by addition of ethylene oxide to sandwich the hydrophobe between hydrophilic groups results in a population of molecules in a relatively circumscribed range of a molecular weights characterized by a hydrophobe having a defined average molecular weight and total average percentage of hydrophile groups.

Since both the ratio and weights of EO and PO vary within this family of surfactants, BASF developed a PLURONIC® grid to provide a graphic representation of the relationship between copolymer structure, physical form and surfactant characteristics. On the PLURONIC^{®} surfactant grid the molecular weight ranges of the hydrophobe (propylene oxide) are plotted against the weight-percent of the hydrophile (ethylene oxide) present in each molecule. Poloxamer species defined by their location on the PLURONIC® grid can be expected to have shared properties that are a function of their total molecular weight and relative hydrophobicity.

The PLURONIC® Grid, a facsimile of which is shown on Figure 18, clarifies the use of the letter-number combinations to identify the various products of the PLURONIC® series. The alphabetical designation explains the physical form of the product: 'L' for liquids, 'P' for pastes, 'F' for solid forms. The first digit (two digits in a three-digit number) in the numerical designation, multiplied by 300, indicates the approximate molecular weight of the hydrophobe (vertical axis at the left of the Grid). The last digit, when multiplied by 10, indicates the approximate ethylene oxide content in the molecule, read from the horizontal axis.

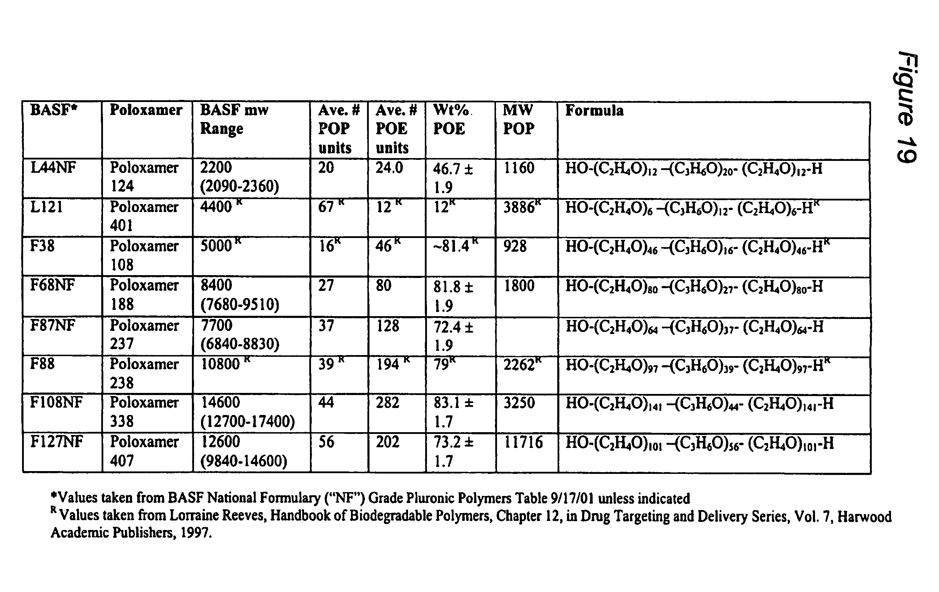

Figure 19 shows the chemical characteristics of poloxamers determined to increase delivery of plasmid DNA to muscle. Preferred poloxamers are circled on Figure 18 and include poloxamers represented by PLURONICS® F38, F68, F87, F88, F108 and F127. In particular, the poloxamer 188, typified by PLURONIC® F68 has been shown by the present inventors to significantly increase the delivery of plasmid DNA with concomitant expression of angiogenic transgenes in both skeletal and cardiac muscle.

### Example 16

Figure 3B and 3C depict the structure of the preferred plasmid vector for use to deliver an angiogenic protein to the ischemic tissue. As shown, the plasmid include post-transcriptional elements (5' UTR including a synthetic intron and hGH polyA signal) that could be expected to improve the level and fidelity of protein expression. In this embodiment, the expression cassette, as shown in Figure 3B, includes a CMV 5' UTR, termed UT12 (SEQ. ID. NO:8) in addition to a synthetic intron, IVS8, within the 5' UTR. The sequence of UT12 (SEQ ID NO: 8) is shown below:

Cryptic splicing in transcripts from eukaryotic expression vectors is obviously undesirable. To obtain control over the splicing pattern and to maximize gene expression, suboptimal introns can be replaced by a strong intron. A synthetic intron with consensus splicing sequences should be optimal for this purpose. The synthetic intron of the present embodiment (IVS 8) includes consensus sequences for the 5' splice site, 3' splice site and branch point. When incorporated into eukaryotic vectors designed to express therapeutic genes, the synthetic intron will direct the splicing of RNA transcripts in a highly efficient and accurate manner, thereby minimizing cryptic splicing and maximizing production of the desired gene product.

The first and sixth position of the 5' splice site consensus sequence are partially ambiguous. The 5' splice site pairs with U1 snRNA. The chosen sequence minimizes the free energy of helix formation between U1 RNA and the synthetic 5' splice site.

In mammals, the branch point sequence is very ambiguous. The branch point sequence, except for a single bulged A residue, pairs with U2 snRNA. The chosen sequence minimizes the free energy of helix formation between U2 RNA and the synthetic branch point sequence. It also matches the branch point sequence that is obligatory for yeast pre-mRNA splicing. The branch point is typically located 18-38 nts upstream of the 3' splice site. The branch point of the synthetic intron is located 24 nts upstream from the 3' splice site.

The polypyrimidine tract of the consensus sequence for 3' splice sites is not exactly defined. At least 5 consecutive uracil residues are needed for optimal 3' splice site function. This concept is incorporated into the polypyrimidine tract of the synthetic intron, which has 7 consecutive uracil residues.

Splicing in vitro is optimal when introns are >80 nts in length. Although many introns may be thousands of bases in length, most naturally occurring introns are 90-200 nt in length. The synthetic intron in the preferred embodiment, IVS8, the length of the synthetic intron is 118 nucleotides. The sequence of IVS8, (SEQ.ID.NO:13), is shown below:

Exonic sequences are in boldface. N = any base. Consensus splicing signals are double-underlined. Restriction enzyme recognition sites are over-lined. The restriction enzyme BbsI may be used to cleave the DNA precisely at the 5' splice site, and EarI may be used to cleave the DNA precisely at the 3' splice site. The two restriction sites, BbsI and EarI, located within the synthetic intron, permit the intron to be easily and precisely deleted. The PstI and NheI sites are included to facilitate the verification of cloning procedures. Double-stranded DNA with this sequence may be prepared using mutually priming long oligonucleotides.

To more closely match the structure of naturally occurring genes, which typically contain many introns, the synthetic intron may be inserted into the gene of interest at multiple locations. When multiple introns are inserted, however, care must be taken to ensure that the lengths of resultant internal exons are less than 300 nucleotides. If internal exons are greater than 300 nucleotides in length, exon skipping may occur.

## Claims

1. A composition comprising a nucleic acid sequence encoding a developmental endothelial locus (del-l) polypeptide and polyglutamate or 1-10% (w/v) Pluronic^{®} F68 (Poloxamer 188) for use in a method of treatment of ischemic conditions in peripheral vascular or coronary artery disease.

2. A composition as claimed in claim 1, wherein the nucleic acid is contained in a plasmid vector.

3. A composition as claimed in claim 1 or claim 2, wherein the nucleic acid encoding the del-I polypeptide comprises SEQ ID NO: 1.

4. A composition as claimed in claim 3, wherein the nucleic acid is a plasmid having SEQ ID NO: 2.

5. A composition as claimed in any preceding claim, wherein the nucleic acid encoding the del-I polypeptide further comprises a CMV promoter, a 5'UTR, a 5' synthetic intron, and a 3'UTR.

6. A composition as claimed in claim 2, consisting of 1 mg/ml plasmid wherein the Pluronic^{®} F68 (Poloxamer 188) is at a concentration of 5% w/v.

7. A composition as claimed in claim 1, wherein the composition is provided as a single vial and is stable at 2-8°C.

8. A composition as claimed in claim 1, wherein the composition is formulated for intramuscular injection or for delivery by retrograde venous perfusion.

9. A composition as claimed in claim 1, wherein the nucleic acid is contained in a plasmid and wherein said plasmid is formulated with Pluronic^{®} F68 (Poloxamer 188) at a concentration of 5% w/v and 5.0mM Tris-HCl buffer.

10. A composition as claimed in claim 9 comprising 5mg of plasmid pDL1680, 250mg of Pluronic^{®} F68 (Poloxamer 188), 0.45 mg of TRIS, and 0.70mg of Tris-HCl.

## Patentansprüche

1. Zusammensetzung, die eine Nukleinsäuresequenz umfasst, die für ein Developmental Endothelial Locus (Del-1) Polypeptid und Polyglutamat codiert oder 1 - 10 % (w/v) Pluronic® F68 (Poloxamer 188), zum Einsatz in einem Verfahren zur Behandlung ischämischer Zustände bei peripheren Gefäßerkrankungen oder Erkrankungen der Herzkranzgefäße.

2. Zusammensetzung nach Anspruch 1, wobei die Nukleinsäure in einem Plasmidvektor enthalten ist.

3. Zusammensetzung nach Anspruch 1 oder Anspruch 2, wobei die Nucleinsäure, die für das DEL-1 Polypeptid codiert, SEQ ID NO: 1 umfasst.

4. Zusammensetzung nach Anspruch 3, wobei es sich bei der Nukleinsäure um ein Plasmid mit SEQ ID NO: 2 handelt.

5. Zusammensetzung nach einem der vorstehenden Ansprüche, wobei die Nukleinsäure, die für das DEL-1 Polypeptid codiert, ferner einen CMV-Promotor, einen 5'UTR, einen 5' synthetischen Intron und einen 3'UTR umfasst.

6. Zusammensetzung nach Anspruch 2, wobei diese 1 mg/nl Plasmid enthält, wobei das Pluronic® F68 (Poloxamer 188) mit einer Konzentration von 5 % w/v vorgesehen ist.

7. Zusammensetzung nach Anspruch 1, wobei die Zusammensetzung als eine einzelne Phiole vorgesehen und bei 2-8 °C stabil ist.

8. Zusammensetzung nach Anspruch 1, wobei die Zusammensetzung für eine intramuskuläre Injektion oder die Verabreichung durch retrograde venöse Perfusion formuliert ist.

9. Zusammensetzung nach Anspruch 1, wobei die Nukleinsäure in einem Plasmid enthalten ist, und wobei das genannte Plasmid mit Plunonic® F68 (Poloxamer 188) mit einer Konzentration von 5 % w/v und einem 5,0 mM Tris-HCI-Puffer formuliert ist,

10. Zusammensetzung nach Anspruch 9, wobei diese 5 mg Plasmid pDL1680, 250 mg Pluronic® F68 (Poloxamer 188), 0,45 mg TRIS und 0,70 mg Tris-HCI umfasst.

## Revendications

1. Composition comprenant une séquence d'acide nucléique codant pour un polypeptide à locus endothélial développemental (duel-1) et du polyglutamate ou 1 à 10 % (p/v) de Pluronic® F68 (Poloxamer 188) destinée à être utilisée dans un procédé de traitement d'affections ischémiques dans une maladie vasculaire périphérique ou coronarienne.

2. Composition selon la revendication 1, dans laquelle l'acide nucléique est contenu dans un vecteur plasmidique.

3. Composition selon la revendication 1 ou la revendication 2, dans laquelle l'acide nucléique codant pour le polypeptide del-1 comprend SEQ ID N° : 1.

4. Composition selon la revendication 3, dans laquelle l'acide nucléique est un plasmide ayant SEQ ID N° : 2.

5. Composition selon l'une quelconque des revendications précédentes, dans laquelle l'acide nucléique codant pour le polypeptide duel-1 comprend en outre un promoteur de CMV, une UTR en 5', un intron synthétique en 5' et une UTR en 3'.

6. Composition selon la revendication 2, constituée de 1 mg/ml de plasmide, dans laquelle le Pluronic® F68 (poloxamer 188) est à une concentration de 5 % p/v.

7. Composition selon la revendication 1, dans laquelle la composition est fournie en tant que fiole unique et est stable à 2 à 8°C.

8. Composition selon la revendication 1, dans laquelle la composition est formulée pour injection intramusculaire ou pour administration par perfusion veineuse rétrograde.

9. Composition selon la revendication 1, dans laquelle l'acide nucléique est contenu dans un plasmide et dans laquelle ledit plasmide est formulé avec du Pluronic® F68 (Poloxamer 188) à une concentration de 5 % p/v et 5,0 mM de tampon Tris-HCI.

10. Composition selon la revendication 9 comprenant 5 mg de plasmide pDL1680, 250 mg de Pluronic® F68 (Poloxamer 188), 0,45 mg de TRIS et 0,70 mg de Tris-HCl.


### SEQUENSE LISTING

<110> VALENTIS, INC.
<120> GENE DELIVERY FORMULATION AND METHOD FOR TREATMENT OF ISCHEMIC CONDITIONS
<130> 265/043
<140> not yet assigned
   <141> 2001-10-19
<150> US 60/242, 277
   <151> 2000-10-20
<150> US 60/294, 454
   <151> 2001-05-29
<160> 13
<170> Patent In version 3.1
<210> 1
   <211> 1443
   <212> DNA
   <213> Homo sapiens
<400> 1
   atgaagcgct cggtagccgt ctggctcttg gtcgggctca gcctcggtgt cccccagttc      60
   ggcaaaggtg atatttgtga tcccaatcca tgtgaaaatg gaggtatctg tttgccagga     120
   ttggctgatg gttccttttc ctgtgagtgt ccagatggct tcacagaccc caactgttct     180
   agtgttgtgg aggttgcatc agatgaagaa gaaccaactt cagcaggtcc ctgcactcct     240
   aatccatgcc ataatggagg aacctgtgaa ataagtgaag cataccgagg ggatacattc     300
   ataggctatg tttgtaaatg tccccgagga tttaatggga ttcactgtca gcacaacata     360
   aatgaatgcg aagttgagcc ttgcaaaaat ggtggaatat gtacagatct tgttgctaac     420
   tattcctgtg agtgcccagg cgaatttatg ggaagaaatt gtcaatacaa atgctcaggc     480
   ccactgggaa ttgaaggtgg aattatatca aaccagcaaa tcacagcttc ctctactcac     540
   cgagctcttt ttggactcca aaaatggtat ccctactatg cacgtcttaa taagaagggg     600
   cttataaatg cgtggacagc tgcagaaaat gacagatggc cgtggattca gataaatttg     660
   caaaggaaaa tgagagttac tggtgtgatt acccaaggag ccaagaggat tggaagccca     720
   gagtatataa aatcctacaa aattgcctac agtaatgatg gaaagacttg ggcaatgtac     780
   aaagtgaaag gcaccaatga agacatggtg tttcgtggaa acattgataa caacactcca     840
   tatgctaact ctttcacacc ccccataaaa gctcagtatg taagactcta tccccaagtt     900
   tgtcgaagac attgcacttt gcgaatggaa cttcttggct gtgaactgtc gggttgttct     960
   gagcctctgg gtatgaaatc aggacatata caagactatc agatcactgc ctccagcatc    1020
   ttcagaacgc tcaacatgga catgttcact tgggaaccaa ggaaagctcg gctggacaag 1080
   caaggcaaag tgaatgcctg gacctctggc cacaatgacc agtcacaatg gttacaggtg 1140
   gatcttcttg ttccaaccaa agtgactggc atcattacac aaggagctaa agattttggt 1200
   catgtacagt ttgttggctc ctacaaactg gcttacagca atgatggaga acactggact 1260
   gtataccagg atgaaaagca aagaaaagat aaggttttcc agggaaattt tgacaatgac 1320
   actcacagaa aaaatgtcat cgaccctccc atctatgcac gacacataag aatccttcct 1380
   tggtcctggt acgggaggat cacattgcgg tcagagctgc tgggctgcac agaggaggaa 1440
   tga 1443
<210> 2
   <211> 5195
   <212> DNA
   <213> Artificial Sequence
<220>
   <223> Sequence of the pDL1680 Human Del-1 Expression Plasmid
<400> 2
   ggtacggtcg ttacataact tacggtaaat ggcccgcctg gctgaccgcc caacgacccc 60
   cgcccattga cgtcaataat gacgtatgtt cccatagtaa cgccaatagg gactttccat 120
   tgacgtcaat gggtggagta tttacggtaa actgcccact tggcagtaca tcaagtgtat 180
   catatgccaa gtacgccccc tattgacgtc aatgacggta aatggcccgc ctggcattat 240
   gcccagtaca tgaccttatg ggactttcct acttggcagt acatctacgt attagtcatc 300
   gctattacca tggtgatgcg gttttggcag tacatcaatg ggcgtggata gcggtttgac 360
   tcacggggat ttccaagtct ccaccccatt gacgtcaatg ggagtttgtt ttggcaccaa 420
   aatcaacggg actttccaaa atgtcgtaac aactccgccc cattgacgca aatgggcggt 480
   aggcgtgtac ggtgggaggt ctatataagc agagctcgtt tagtgaaccg tcagatcgcc 540
   tggagacgcc atccacgctg ttttgacctc catagaagac accgggaccg atccagcctc 600
   cgcggccggg aacggtgcat tggaacgcgg attccccgtg ttaattaaca ggtaagtgtc 660
   ttcctcctgt ttccttcccc tgctattctg ctcaaccttc ctatcagaaa ctgcagtatc 720
   tgtatttttg ctagcagtaa tactaacggt tctttttttc tcttcacagg ccaccaagct 780
   tatgaagcgc tcggtagccg tctggctctt ggtcgggctc agcctcggtg tcccccagtt 840
   cggcaaaggt gatatttgtg atcccaatcc atgtgaaaat ggaggtatct gtttgccagg 900
   attggctgat ggttcctttt cctgtgagtg tccagatggc ttcacagacc ccaactgttc 960
   tagtgttgtg gaggttgcat cagatgaaga agaaccaact tcagcaggtc cctgcactcc 1020
   taatccatgc cataatggag gaacctgtga aataagtgaa gcataccgag gggatacatt 1080
   cataggctat gtttgtaaat gtccccgagg atttaatggg attcactgtc agcacaacat   1140
   aaatgaatgc gaagttgagc cttgcaaaaa tggtggaata tgtacagatc ttgttgctaa   1200
   ctattcctgt gagtgcccag gcgaatttat gggaagaaat tgtcaataca aatgctcagg   1260
   cccactggga attgaaggtg gaattatatc aaaccagcaa atcacagctt cctctactca   1320
   ccgagctctt tttggactcc aaaaatggta tccctactat gcacgtctta ataagaaggg   1380
   gcttataaat gcgtggacag ctgcagaaaa tgacagatgg ccgtggattc agataaattt   1440
   gcaaaggaaa atgagagtta ctggtgtgat tacccaagga gccaagagga ttggaagccc   1500
   agagtatata aaatcctaca aaattgccta cagtaatgat ggaaagactt gggcaatgta   1560
   caaagtgaaa ggcaccaatg aagacatggt gtttcgtgga aacattgata acaacactcc   1620
   atatgctaac tctttcacac cccccataaa agctcagtat gtaagactct atccccaagt   1680
   ttgtcgaaga cattgcactt tgcgaatgga acttcttggc tgtgaactgt cgggttgttc   1740
   tgagcctctg ggtatgaaat caggacatat acaagactat cagatcactg cctccagcat   1800
   cttcagaacg ctcaacatgg acatgttcac ttgggaacca aggaaagctc ggctggacaa   1860
   gcaaggcaaa gtgaatgcct ggacctctgg ccacaatgac cagtcacaat ggttacaggt   1920
   ggatcttctt gttccaacca aagtgactgg catcattaca caaggagcta aagattttgg   1980
   tcatgtacag tttgttggct cctacaaact ggcttacagc aatgatggag aacactggac   2040
   tgtataccag gatgaaaagc aaagaaaaga taaggttttc cagggaaatt ttgacaatga   2100
   cactcacaga aaaaatgtca tcgaccctcc catctatgca cgacacataa gaatccttcc   2160
   ttggtcctgg tacgggagga tcacattgcg gtcagagctg ctgggctgca cagaggagga   2220
   atgagggtat actacgaccg tctagaaaag ccgaattctg caggaattgg gtggcatccc   2280
   tgtgacccct ccccagtgcc tctcctggcc ctggaagttg ccactccagt gcccaccagc   2340
   cttgtcctaa taaaattaag ttgcatcatt ttgtctgact aggtgtcctt ctataatatt   2400
   atggggtgga ggggggtggt atggagcaag gggcaagttg ggaagacaac ctgtagggct   2460
   cgaggggggg cccggtactc tgccggtctc cctatagtga gtcgtattaa tttcgataag   2520
   ccaggttaac ctgcattaat gaatcggcca acgcgcgggg agaggcggtt tgcgtattgg   2580
   gcgctcttcc gcttcctcgc tcactgactc gctgcgctcg gtcgttcggc tgcggcgagc   2640
   ggtatcagct cactcaaagg cggtaatacg gttatccaca gaatcagggg ataacgcagg   2700
   aaagaacatg tgagcaaaag gccagcaaaa ggccaggaac cgtaaaaagg ccgcgttgct   2760
   ggcgtttttc cataggctcc gcccccctga cgagcatcac aaaaatcgac gctcaagtca   2820
   gaggtggcga aacccgacag gactataaag ataccaggcg tttccccctg gaagctccct   2880
   cgtgcgctct cctgttccga ccctgccgct taccggatac ctgtccgcct ttctcccttc   2940
   gggaagcgtg gcgctttctc atagctcacg ctgtaggtat ctcagttcgg tgtaggtcgt 3000
   tcgctccaag ctgggctgtg tgcacgaacc ccccgttcag cccgaccgct gcgccttatc 3060
   cggtaactat cgtcttgagt ccaacccggt aagacacgac ttatcgccac tggcagcagc 3120
   cactggtaac aggattagca gagcgaggta tgtaggcggt gctacagagt tcttgaagtg 3180
   gtggcctaac tacggctaca ctagaagaac agtatttggt atctgcgctc tgctgaagcc 3240
   agttaccttc ggaaaaagag ttggtagctc ttgatccggc aaacaaacca ccgctggtag 3300
   cggtggtttt tttgtttgca agcagcagat tacgcgcaga aaaaaaggat ctcaagaaga 3360
   tcctttgatc ttttctacgg ggtctgacgc tcagtggaac gaaaactcac gttaagggat 3420
   tttggtcatg agcgcgccta ggcttttgca aagatcgatc aagagacagg atgaggatcg 3480
   tttcgcatga ttgaacaaga tggattgcac gcaggttctc cggccgcttg ggtggagagg 3540
   ctattcggct atgactgggc acaacagaca atcggctgct ctgatgccgc cgtgttccgg 3600
   ctgtcagcgc aggggcgccc ggttcttttt gtcaagaccg acctgtccgg tgccctgaat 3660
   gaactgcaag acgaggcagc gcggctatcg tggctggcca cgacgggcgt tccttgcgca 3720
   gctgtgctcg acgttgtcac tgaagcggga agggactggc tgctattggg cgaagtgccg 3780
   gggcaggatc tcctgtcatc tcaccttgct cctgccgaga aagtatccat catggctgat 3840
   gcaatgcggc ggctgcatac gcttgatccg gctacctgcc cattcgacca ccaagcgaaa 3900
   catcgcatcg agcgagcacg tactcggatg gaagccggtc ttgtcgatca ggatgatctg 3960
   gacgaagagc atcaggggct cgcgccagcc gaactgttcg ccaggctcaa ggcgagcatg 4020
   cccgacggcg aggatctcgt cgtgacccat ggcgatgcct gcttgccgaa tatcatggtg 4080
   gaaaatggcc gcttttctgg attcatcgac tgtggccggc tgggtgtggc ggaccgctat 4140
   caggacatag cgttggctac ccgtgatatt gctgaagagc ttggcggcga atgggctgac 4200
   cgcttcctcg tgctttacgg tatcgccgct cccgattcgc agcgcatcgc cttctatcgc 4260
   cttcttgacg agttcttctg agcgggactc tggggttcga aatgaccgac caagcgacgc 4320
   ccaacctgcc atcacgagat ttcgattcca ccgccgcctt ctatgaaagg ttgggcttcg 4380
   gaatcgtttt ccgggacgcc ggctggatga tcctccagcg cggggatctc atgctggagt 4440
   tcttcgccca ccctaggcgc gctcatgagc ggatacatat ttgaatgtat ttagaaaaat 4500
   aaacaaatag gggttccgcg cacatttccc cgaaaagtgc cacctaaatt gtaagcgtta 4560
   atattttgtt aaaattcgcg ttaaattttt gttaaatcag ctcatttttt aaccaatagg 4620
   ccgaaatcgg caaaatccct tataaatcaa aagaatagac cgagataggg ttgagtgttg 4680
   ttccagtttg gaacaagagt ccactattaa agaacgtgga ctccaacgtc aaagggcgaa 4740
   aaaccgtcta tcagggcgat ggcccactac gtgaaccatc accctaatca agttttttgg 4800
   ggtcgaggtg ccgtaaagca ctaaatcgga accctaaagg gagcccccga tttagagctt 4860
   gacggggaaa gccggcgaac gtggcgagaa aggaagggaa gaaagcgaaa ggagcgggcg 4920
   ctagggcgct ggcaagtgta gcggtcacgc tgcgcgtaac caccacaccc gccgcgctta 4980
   atgcgccgct acagggcgcg tcccattcgc cattcaggct gcgcaactgt tgggaagggc 5040
   gatcggtgcg ggcctcttcg ctattacgcc agctggcgaa agggggatgt gctgcaaggc 5100
   gattaagttg ggtaacgcca gggttttccc agtcacgacg ttgtaaaacg acggccagtg 5160
   agcgcgcgta atacgactca ctatagggcg aattg 5195
<210> 3
   <211> 576
   <212> DNA
   <213> Artificial Sequence
<220>
   <223> Codon optimized VEGF 165
<400> 3
   atgaactttc tgctgtcttg ggtgcattgg agcctggccc tgctgctcta cctccaccat 60
   gccaagtggt cccaggctgc acccatggca gaaggaggag ggcagaatca tcacgaagtg 120
   gtgaagttca tggatgtcta tcagcgcagc tactgccatc caatcgagac cctggtggac 180
   atcttccagg agtaccctga tgagatcgag tacatcttca agccatcctg tgtgcccctg 240
   atgcgctgcg ggggctgctg caatgacgag ggcctggagt gtgtgcccac tgaggagtcc 300
   aacatcacca tgcagattat gcggatcaaa cctcaccaag gccagcacat cggagagatg 360
   agcttcctgc agcacaacaa atgtgaatgc agaccaaaga aagatagagc aagacaagaa 420
   aatccctgtg ggccttgctc cgagcggaga aagcatctgt ttgtgcaaga tccgcagacg 480
   tgtaaatgtt cctgcaaaaa cacagactcg cgttgcaagg cgaggcagct tgagttaaac 540
   gaacgtactt gcagatgtga caagccgagg cggtga 576
<210> 4
   <211> 25
   <212> DNA
   <213> Artificial sequence
<220>
   <223> Del-1 sense PCR primer
<400> 4
   tgacctccat agaagacacc gggac 25
<210> 5
   <211> 25
   <212> DNA
   <213> Artificial sequence
<220>
   <223> Del-1 anti-sense PCR primer
<400> 5
   gtgatgcaac ctccacaaca ctaga 25
<210> 6
   <211> 19
   <212> DNA
   <213> Artificial sequence
<220>
   <223> VEGF anti-sense PCR primer
<400> 6
   ggaggggtca cagggatgc 19
<210> 7
   <211> 576
   <212> DNA
   <213> Homo sapiens
<400> 7
   atgaactttc tgctgtcttg ggtgcattgg agccttgcct tgctgctcta cctccaccat 60
   gccaagtggt cccaggctgc acccatggca gaaggaggag ggcagaatca tcacgaagtg 120
   gtgaagttca tggatgtcta tcagcgcagc tactgccatc caatcgagac cctggtggac 180
   atcttccagg agtaccctga tgagatcgag tacatcttca agccatcctg tgtgcccctg 240
   atgcgatgcg ggggctgctg caatgacgag ggcctggagt gtgtgcccac tgaggagtcc 300
   aacatcacca tgcagattat gcggatcaaa cctcaccaag gccagcacat aggagagatg 360
   agcttcctac agcacaacaa atgtgaatgc agaccaaaga aagatagagc aagacaagaa 420
   aatccctgtg ggccttgctc agagcggaga aagcatttgt ttgtacaaga tccgcagacg 480
   tgtaaatgtt cctgcaaaaa cacagactcg cgttgcaagg cgaggcagct tgagttaaac 540
   gaacgtactt gcagatgtga caagccgagg cggtga 576
<210> 8
   <211> 110
   <212> DNA
   <213> Artificial sequence
<220>
   <223> 5' Untranslated region derived from CMV termed UT12
<400> 8
   tcagatcgcc tggagacgcc atccacgctg ttttgacctc catagaagac accgggaccg 60
   atccagcctc cgcggccggg aacggtgcat tggaacgcgg attccccgtg 110
<210> 9
   <211> 9
   <212> RNA
   <213> Artificial sequence
<220>
   <223> 5' splice site sequence of a synthetic intron
<400> 9
   cagguaagu 9
<210> 10
   <211> 9
   <212> RNA
   <213> Homo sapiens
<400> 10
   guccauuca 9
<210> 11
   <211> 7
   <212> RNA
   <213> Artificial sequence
<220>
   <223> Branch point sequence for a synthetic intron
<400> 11
   uacuaac 7
<210> 12
   <211> 6
   <212> RNA
   <213> Homo sapiens
<400> 12
   augaug 6
<210> 13
   <211> 160
   <212> DNA
   <213> Artificial sequence
<220>
   <223> A synthetic intron termed IVS8 where exonic sequences are represented by n, n can be any base and can be longer or shorter
<220>
   <221> misc_feature
   <222> (1)..(10)
   <223> any base
<220>
   <221> misc_feature
   <222> (151)..(160)
   <223> any base
<400> 13
   nnnnnnnnnn ttaattaaca ggtaagtgtc ttcctcctgt ttccttcccc ctgctattct     60
   gctcaacctt cctatcagaa actgcagtat ctgtattttt gctagcagtt atactaacgg    120
   ttcttttttt ctcttcacag gccaccatgg nnnnnnnnnn                          160